# EUROPEAN PATENT APPLICATION

(11) **EP 2 108 654 A1**
(43) Date of publication of application: **14.10.2009**
(21) Application number: 08103412.6
(22) Date of filing: 07.04.2008
(51) Int. Cl.: C07K 1/36, C07K 1/14

(54) **Selective enrichment of n-terminally modified peptides from complex samples**

(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The present invention relates to methods allowing selective enrichment of N-terminal fragments of polypeptides and/or peptides from complex samples by combining a particular polypcptidc/pcptidc labeling and fractionation strategy with specific chemical and/or enzymatic reactions targeting the N-terminal fragments to be analyzed

## Description

### SUBJECT OF THE INVENTION

The present invention relates to methods allowing selective enrichment ofN-terminal fragments of polypeptides and/or peptides from complex samples by combining a particular polypeptide/peptide labeling and fractionation strategy with specific chemical and/or enzymatic reactions targeting N-terminal fragments to be analyzed.

### BACKGROUND OF THE INVENTION

The identification, separation, and analysis of particular proteins or subsets of proteins from complex samples are invaluable for unraveling how biological processes occur at a molecular level or to which degree proteins differ among various cell types or between physiological states.

A major challenge in modem biology is directed to the understanding of the expression, function, and regulation of the entire set of proteins encoded by an organism, a technical field commonly known as proteomics. However, as there is no possibility to amplify proteins, research in this field is generally rather tedious because even a cell extract of a relatively simple prokaryotic organism contains a multitude of proteins encompassing a huge range of concentrations. Therefore, such a task is beyond the capabilities of any current single analytical methods.

Thus, due to the methodological constraints proteome analysis relies not only to methods for identifying and quantifying proteins but - to a considerable extent - also on methods allowing their accurate and reliable separation according to their structural and/or functional properties, with these subsets being then better accessible to further analysis.

The proteome is of dynamic nature, with alterations in protein synthesis, activation, and/or post-translational modification in response to external stimuli or alterations in the cellular environment. Therefore, the proteome's inherent complexity exceeds that of the genome or the transcriptome the mRNA complement of a cell.

Due to the extraordinary amount of data to be processed in such proteomic studies the protein/peptide identification process demands tremendous resolving power. Two methods commonly used to resolve such highly complex mixtures are two-dimensional gel electrophoresis (2D-GE; cf., e.g., O'Farrell, P.H. (1975) J. Biol. Chem. 250, 4007-4021) and (two-dimensional) liquid chromatography ((2D)-LC; cf., e.g., Lipton, M.S. et al. (2002) Proc. Natl. Acad. Sci. USA 99, 11049-11054). The peptides and proteins isolated by 2D-GE or 2D-LC are usually identified by mass spectrometry or by determining amino acid composition and/or amino acid sequence.

Mass spectrometry in particular allows in principle for accurate qualitative identification and quantitative determination of proteins in a high throughput setup. Nevertheless, it suffers from some inherent technical limitations. One of the biggest problems nowadays in the mass spectrometry based analysis of proteins is the dynamic range and number of proteins to be detected. Particularly in samples like serum or plasma, the dynamic range is estimated to be in the order of 10¹² in combination with several 100,000's of proteins. Typical workflows for identifying proteins and proteomics involve proteomic digestion of these proteins and the analysis of the resulting fragments. In the case of human plasma or serum this can increase the complexity by roughly a factor of 50 given that 55 tryptic peptides are generated on average when plasma or serum proteins are digested with trypsin. At the same time, the dynamic range of most mass spectrometers is not more than 100 in a single spectrum, so that the available separation window either does not allow detection of all proteins present or must be increased for that purpose.

Different approaches have been undertaken in the art to reduce the complexity by labeling the proteins to be detected and to isolate only the labeled peptide fragments resulting from e.g. a trypsic digest before mass spectrometry analysis is undertaken. The rationale beyond this approach is that the labeling efficiency will be comparable equal across the vast majority of proteins to be detected so that the analysis of labeled peptide fragments only will give a representative picture of the proteins being present in a sample to be analyzed.

Even though such processes have allowed reduction of the complexity of signals to be detected in a proteomic analysis, a continuing need exists for further approaches to purposively select certain subsets of proteins and peptides for mass spectrometry analysis.

### OBJECT AND SUMMARY OF THE INVENTION

It is one objective of the present invention to provide novel approaches for the selective enrichment of peptide fragments of polypeptides and/or peptides from complex samples for use in mass spectrometry analysis. More specifically, it is an objective of the present invention to provide methods that allow for selective enrichment of peptide fragments of polypeptides and/or peptides to be analyzed in parallel by mass spectrometry.

These objectives as well as others which will become apparent from the ensuing description are attained by the subject matter of the independent claims. Some of the preferred embodiments of the present invention are defined by the subject matter of the dependent claims.

In one embodiment, the present invention relates to a method for the selective enrichment and/or separation ofN-terminal fragments of polypeptides and/or peptides in a sample, comprising:
(a) labeling of proteins, polypeptides and/or peptides comprised within at least a first sample with at least one first label;
(b) labeling of proteins, polypeptides and/or peptides comprised within at least a second sample with at least one second label;
(c) combining the labeled proteins, polypeptides and/or peptides of steps (a) and (b);
(d) digesting the labeled proteins, polypeptides and/or peptides to generate fragments thereof;
(e) fractionating said fragments;
(f) performing a methylation reaction with said fragments;
(g) re-fractionating said fragments;
(h) comparing the fractionation patterns obtained in steps (e) and (g); and

separating N-terminal fragments of the polypeptides and/or peptides of steps (a) and (b) which carry a label based on the results obtained in step (g).

In a preferred embodiment the fractionation/refractionation of steps (e) and (g) is performed via isoelectric focusing.

In another preferred embodiment, the at least one label is configured to allow labeling of primary amines of said proteins, polypeptides and/or peptides.

In yet another preferred embodiment, the labeling of the proteins, polypeptides and/or peptides comprised in the sample will include labeling with at least two different labels. The at least two different labels may preferably be isotopic labels.

In yet a further preferred embodiment of the present invention, said at least two different labels may be isobaric labels.

In yet another further preferred embodiment of the present invention, said at least two different labels may be isotopic labels which further comprise isobaric labels.

In a preferred embodiment, the labels of steps a) and b) are used to label the proteins, polypeptides and/or peptides within the respective samples at all available free amine groups as they occur e.g. at the N-terminus and in the amino acids lysine and arginine.

Another preferred embodiment of the present invention includes a methylation reaction in step (f) allowing for methylation of amine groups.

One embodiment of the present invention includes the further analysis of the N-terminal peptide fragments identified in step (g) by mass spectrometry. Such mass spectrometry analysis may include an MS/MS scan. One embodiment of the present invention relates to the method being performed in a high throughput format.

The methods of the present invention may be used for performing qualitative and/or quantitative proteomic analysis.

Other embodiments of the present invention will become apparent from the detailed description hereinafter.

### FIGURE LEGENDS

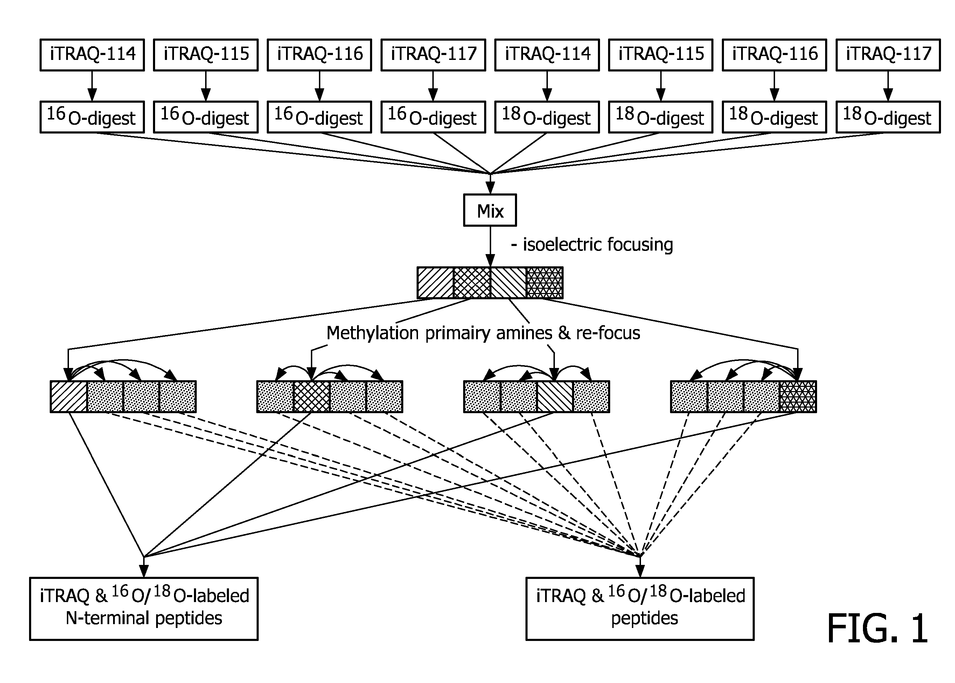

- Figure 1:: Depicts a schematic illustration of the application of a preferred embodiment of the invention to allow for selective enrichment of N-terminal peptide fragments of proteins, polypeptides or peptides. The e.g. polypeptides comprised in a given sample are digested in the presence of either ¹⁶O or ¹⁸O-labelled water (isotopic labeling). Before the isotopic labeling, the polypeptides or resulting fragments are labeled using an isobaric label for relative and absolute quantitation technology (iTraq, isobaric labeling) which is used in amount to label all available amine groups within the polypeptides of the sample. The labeled fragments are fractionated by isoelectric focusing (IEF). All individual fractions are collected and individually treated in a reaction to methylate N-terminal amine groups. All fractions are then individually re-fractionated by IEF. Peptides sorted to the same fraction as after the first round of IEF have not undergone any additional modification by methylation and are thus N-terminal fragments that have originally been labeled with the iTraq marker. However, peptides sorted to a different fraction of the re-fractionation have undergone methylation at their N-terminus which became available after digestion. The comparison of the first fractionation step and the re-fractionation step thus allows for identification of those peptides that are N-terminal fragments of polypeptides and/or peptides.

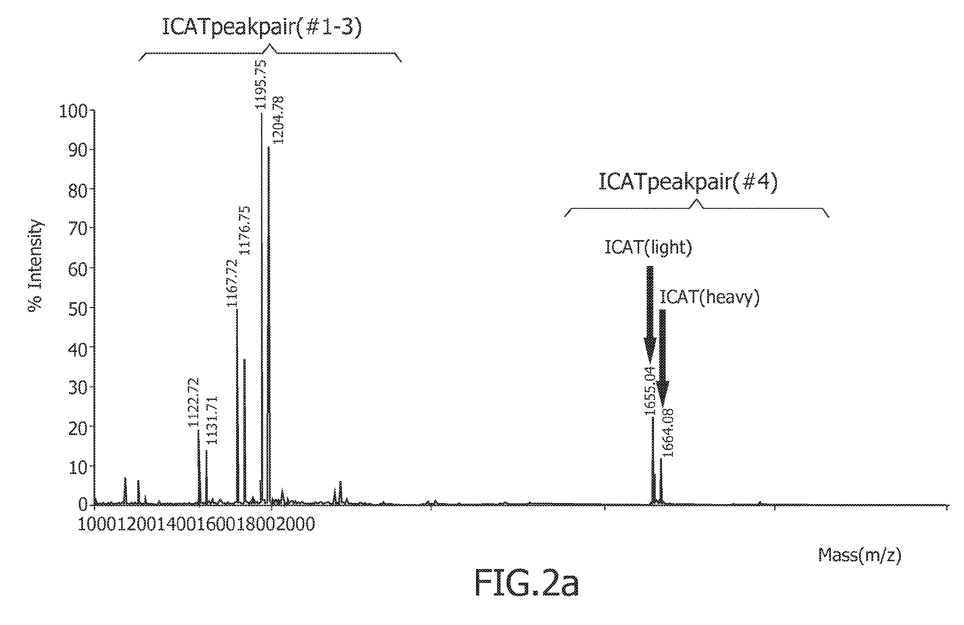

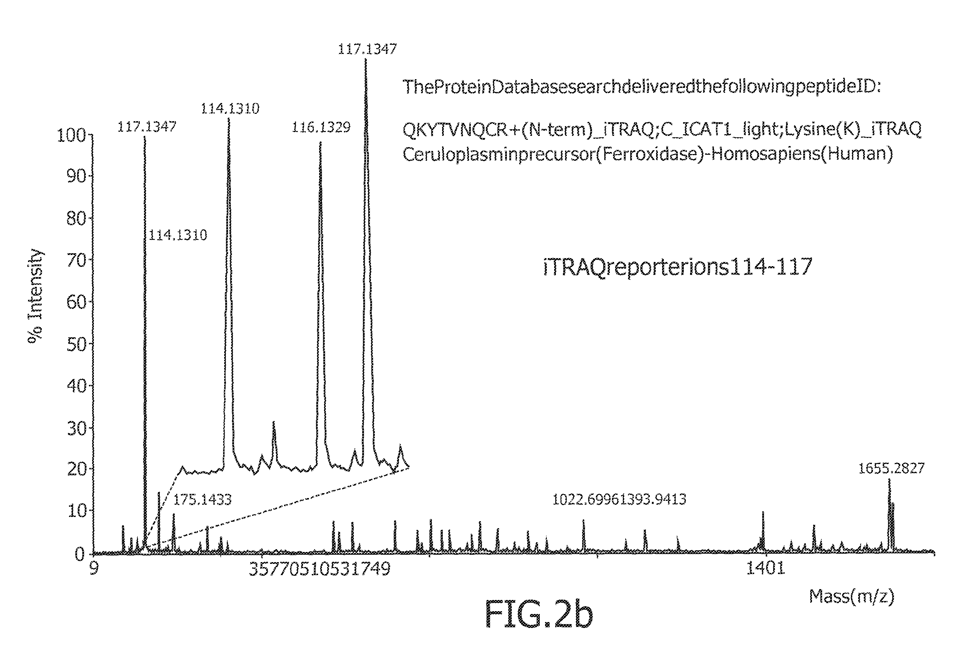

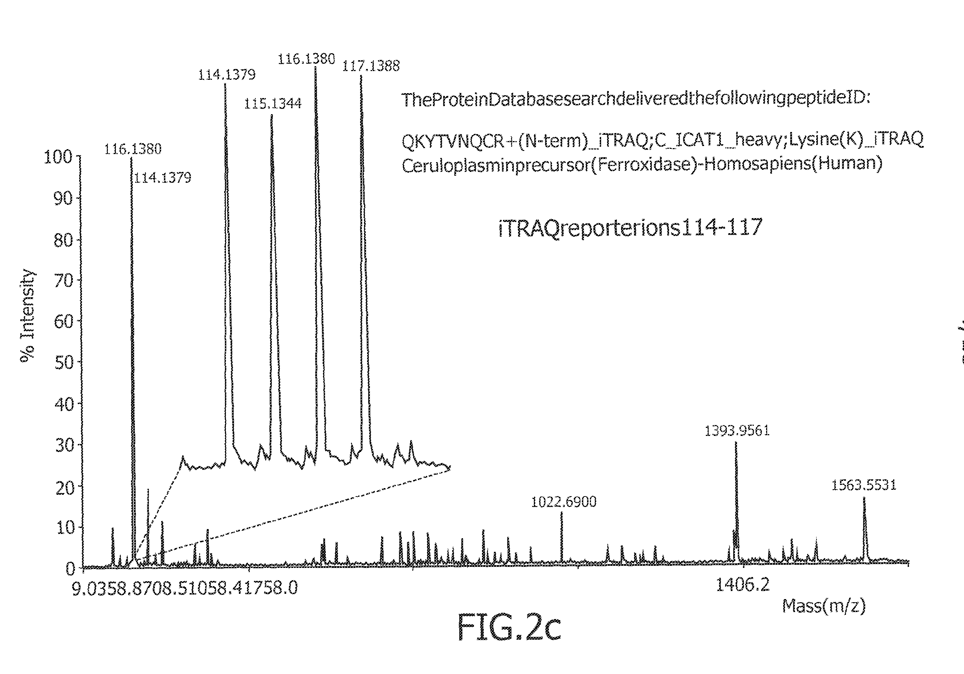

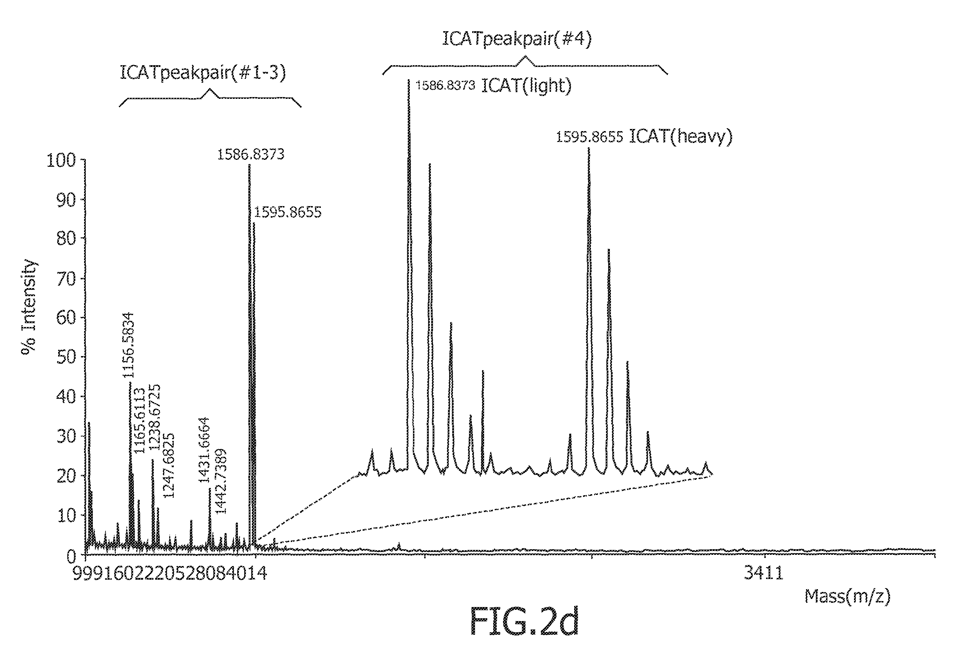

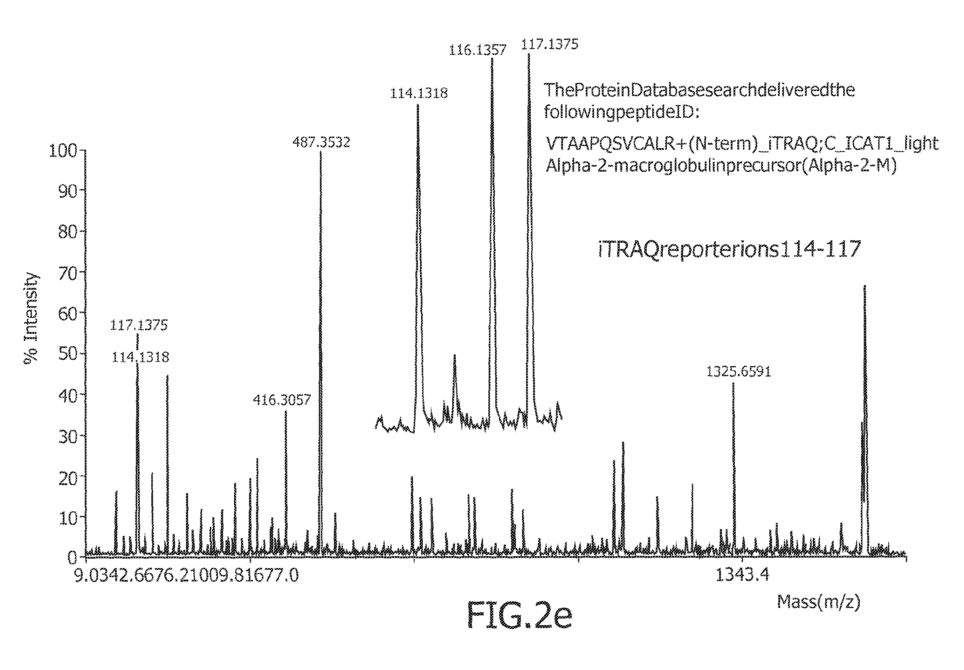

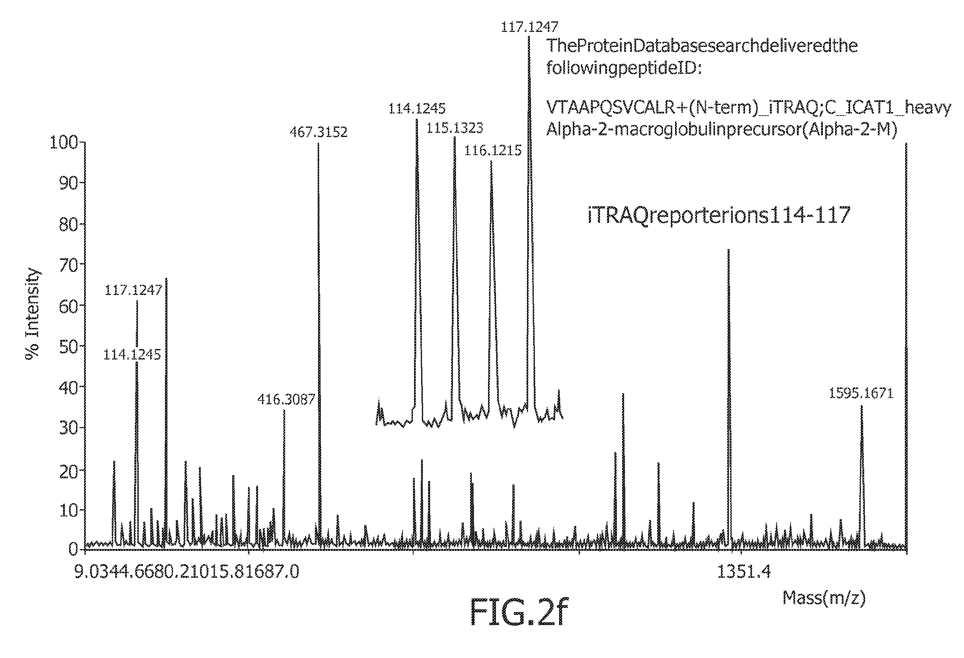

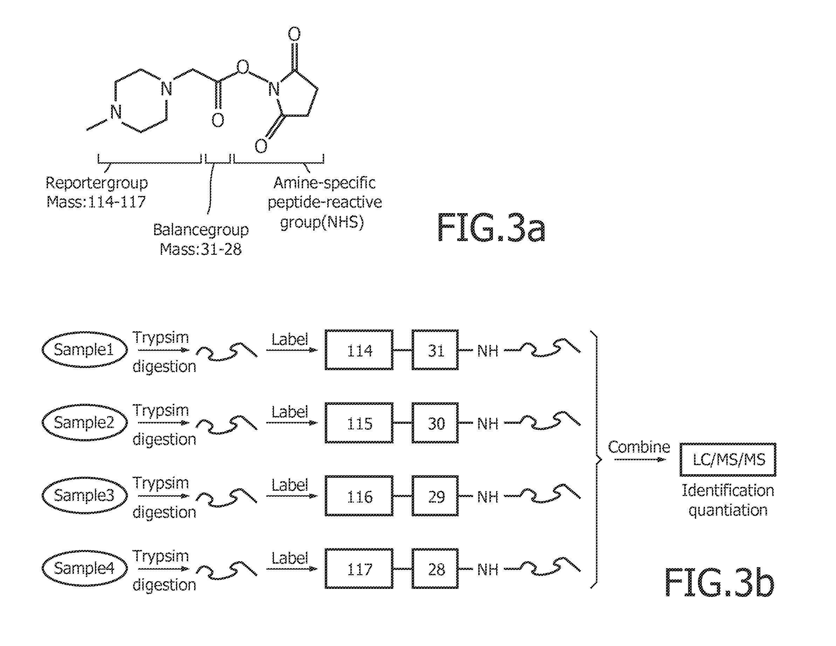

- Figure 2:: Depicts the results of a MALDI-MS and MALDI-MS/MS analysis using double chemical labeled peptides comprised in an immuno-depleted plasma sample. The plasma sample was immuno-depleted by means of a Multiple Affinity Removal LC Column - Human 7 (Agilent Technologies, Inc., Santa Clara, CA, USA). The proteins were isotopically labeled using the ICAT reagent (Applied Biosystems, Inc., Foster City, CA, USA) essentially according to the manufacturer's instructions and digested with trypsin (ratio trypsin:sample = 1:20). Subsequently, the resulting peptides were isobarically labeled essentially following the established iTRAQ protocol (Applied Biosystems, Inc., Foster City, CA, USA). The labeled peptides were processed by strong cation exchange (SCX) fractionation using 50-70-90-100-110-120-130-140-150-180-210-350 mM KCl/ 10mM KH₂PO₄/25% acetonitrile, pH 3.0 and RP-HPLC (20 s fractions, 380 fractions per SCX fraction). MALDI-MS and MALDI-MS/MS analysis were performed on 4579 spots. **Fig.2a**shows a MS analysis of RP-HPLC fraction #12 (SCX fraction 350 mM KCl). Four ICAT peak pairs have been assigned: the expected peak ratio between ICAT(light): ICAT(heavy) = 1.8. **Fig. 2b**shows a MS/MS analysis of ICAT(light) (1655.04 Da) of peak pair #4 (see Fig. 3A). The iTRAQ reporter m/z region (114-117) is enlarged; the expected iTRAQ ratios are: 1:0.1:1:1 **Fig. 2c**shows a MS/MS analysis of ICAT(heavy) (1664.08 Da) of peak pair #4 (see Fig. 3A). The iTRAQ reporter m/z region (114-117) is enlarged; the expected iTRAQ ratios are: 1:1:1:1. **Fig. 2d**shows a MS analysis of RP-HPLC fraction #39 (SCX fraction 50 mM KCL). Four ICAT peak pairs have been assigned; the expected peak ratio between ICAT(light):ICAT(heavy) = 1.5. **Fig. 2e**shows a MS/MS analysis of ICAT(light) (1586.84 Da) of peak pair #4 (see Fig. 3D). The iTRAQ reporter m/z region (114-117) is enlarged; the expected iTRAQ ratios are: 1:0.1:1:1. **Fig. 2f**shows a MS/MS analysis of ICAT(heavy) (1595.87 Da) of peak pair #4 (see Fig. 3D). The iTRAQ reporter m/z region (114-117) is enlarged; the expected iTRAQ ratios are: 1:1:1:1.
- Figure 3:: Depicts the general structure of an isobaric label comprising a reporter and a balance group.
- Figure 4:: Depicts a set of isotopic labels all using a poly-Leu-tag as affinity group. The labels differ e.g. in the use of ¹²C and ¹³C.
- Figure 5:: depicts the detailed reaction steps for the chemical synthesis of the (Leu)₃ compound shown in Fig. 4a). The synthesis protocol is outlined in Example 2.

### DETAILED DESCRIPTION OF THE INVENTION

The present invention is based on the unexpected finding that combining a particular protein/polypeptide/peptide labeling and fractionation strategy with specific chemical and/or enzymatic reactions targeting primary amines allows the rapid and highly selective enrichment and/or separation of N-terminal fragments of these polypeptides/peptides from a complex sample.

The present invention as illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings as described are only schematic and nonlimiting. In the drawings, the size of some of the elements may be exaggerated and not drawn on scale for illustrative purposes.

Where the term "comprising" is used in the present description and claims, it does not exclude other elements. For the purposes of the present invention, the term "consisting of" is considered to be a preferred embodiment of the term "comprising of'. If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. The terms "about" or "approximately" in the context of the present invention, denote an interval of accuracy that the person skilled in the art will understand to still ensure the technical effect of the feature in question. The term typically indicates deviation from the indicated numerical value of ±10%, and preferably of ±5%.

Further definitions of terms will be given in the following in the context of which the terms are used.

In one embodiment, the present invention relates to a method for the selective enrichment and/or separation ofN-terminal fragments of polypeptides and/or peptides in a sample, comprising:
(a) labeling of proteins, polypeptides and/or peptides comprised within at least a first sample with at least one first label;
(b) labeling of proteins, polypeptides and/or peptides comprised within at least a second sample with at least one second label;
(c) combining the labeled proteins, polypeptides and/or peptides of steps (a) and (b);
(d) digesting the labeled proteins, polypeptides and/or peptides to generate fragments thereof;
(e) fractionating said fragments;
(f) performing a methylation reaction with said fragments;
(g) re-fractionating said fragments;
(h) comparing the fractionation patterns obtained in steps (e) and (g); and
(i) separating N-terminal fragments of the polypeptides and/or peptides of steps (a) and (b) which carry a label based on the results obtained in step (h).

The term "proteins" as used herein, refers to any naturally occurring or synthetic (e.g. generated by chemical synthesis or recombinant DNA technology) macromolecule comprising a plurality of natural or modified amino acids connected via peptide bonds. The term "polypeptides" similarly refers to naturally occurring or synthetic macromolecules comprising a plurality of natural or modified amino acids connected by a peptide bond. The length of such molecules may extend up to several thousand amino acids. The term thus also includes what is generally referred to as oligopeptides. Typically, the term "proteins" or "polypeptides" relates to molecules having a length of more than 20 amino acids. Thus, proteins to be analyzed in the present invention may have a length from about 30 to about 500 amino acids, from about 50 to about 1,000 amino acids or from about 100 to about 2,000 amino acids, etc..

The term "peptide" as used herein refers to a naturally occurring or synthetic molecule comprising no more than 20 natural or modified amino acids connected via peptide bond. Typically, peptides to be analysed in the present invention may have a length from about 2 to about 20 amino acids, from about 3 to about 18 amino acids or from about 5 to about 15 amino acids.

The term "fragment" as used herein refers to any fragments of the above mentioned proteins, polypeptides and peptides that are obtained after cleavage of one or more peptide bonds. The cleavage as will be set out hereinafter may be performed chemically and/or enzymatically. A fragment as used in the present invention is not limited in any way with regard to its size or nature except that it is shorter in terms of its amino acid sequence than the proteins, polypeptides or peptides from which it was derived.

The term "N-terminal fragment", as used herein, denotes such fragments of proteins, polypeptides or peptides which are located closest to the N-terminus of the aforementioned proteins, polypeptides or peptides as they are obtainable by the chemical and/or enzymatic digestion in step (b).

The term "post-translational modification", as used herein, denotes any type of chemical modification of a protein, polypeptide and/or peptide according to the invention that takes place after completion of protein translation. Examples of such modifications include *inter alia* phosphorylation, ubiquitinylation, acetylation, glycosylation, alkylation, isoprenylation, and lipoylation, with phosphorylation being particularly preferred. The term is also to be understood not to be limited with regard to the numbers and/of types of post-translational modifications being comprised in a protein and/or peptide. Thus, a given protein may comprise in its sequence two or more phosphorylated amino acids or one or more phosphorylated amino acids and one or more ubiquitinylated amino acid residues.

The terms "phospho-proteins", "phosphor-polypeptides" and "phospho-peptides", as used herein, denote any proteins, polypeptides and/or peptides comprising in their primary sequence one or more phosphorylated amino acid residues, particularly phosphorylated serine, threonine or tyrosine residues. Within the scope of the present invention, amino acid phosphorylation may occur *in vivo* by post-translational protein modification or *in nitro* by employing specific protein kinases.

The terms "glycosylated proteins" (herein also referred to as "glyco-proteins"), "glycosylated polypeptides" (herein also referred to as "glyco-polypeptides") and "glycosylated peptides" (herein also referred to as "glyco-peptides"), as used herein, denote any proteins, polypeptides and/or peptides comprising in their primary sequence one or more glycosylated amino acid residues, wherein the glycosylation may be an *N-*glycosylation, an *O*-glycosylation or a glycosylphosphatidylinisotol-anchoring. The term "*N*-glycosylation" (herein also referred to "*N*-linked glycosylation"), as used herein, denotes the enzyme-directed and site-specific addition of any saccharide moiety (i.e. a carbohydrate or sugar moiety including monosaccharides such as glucose or galactose, disaccharides such as maltose and sucrose and oligo- or polysaccharides) to the amide nitrogen of asparagine amino acid residues, while the term "O-glycosylation" (herein also referred to "O-linked glycosylation"), as used herein, refers to the enzyme-directed and site-specific addition of any saccharide moiety to the hydroxy oxygen of serine or threonine amino acid residues. Finally, the term "glycosylphosphatidylinisotol-anchoring" (herein also referred to "GPI-anchoring"), as used herein, denotes the addition of a hydrophobic phosphatidylinositol group linked through a carbohydrate containing linker (such as glucosamine and mannose linked to a phosphoryl ethanolamine residue) to the C-terminal amino acid of a protein and/or peptide, wherein the two fatty acids within the phosphatidylinositol group anchor the protein to the cell membrane. Within the scope of the present invention, amino acid glycosylation may occur *in vivo* by post-translational protein modification or *in vitro* by employing specific glycosyl transferases.

The terms "sample" or "complex sample", as used herein, denotes the fact that a sample analyzed using a method of the present invention typically includes a multitude of different proteins, polypeptides and/or peptides (or different variants of such proteins, polypeptides and/or peptides) present in different concentrations. For example, complex samples within the present invention may include at least about 500, at least about 1000, at least about 5000 or at least about 10000 proteins, polypeptides and/or peptides. Typical complex samples used in the invention include *inter alia* cell extracts or lysates of prokaryotic or eukaryotic origin as well as human or non-human body fluids such as whole blood, serum, plasma samples or the like.

In methods in accordance with the invention, proteins, polypeptides and/or peptides may be chemically labeled.

The term "chemical labeling", as used herein, denotes the attachment or incorporation of one or more detectable markers (or "labels") into a protein, polypeptide and/or peptide used in the invention. The term "detectable marker", as used herein, refers to any compound that comprises one or more appropriate chemical substances or enzymes, which directly or indirectly generate a detectable compound or signal in a chemical, physical or enzymatic reaction. As used herein, the term is to be understood to include both the labels as such (i.e. the compound or moiety bound to the protein and/or peptide) as well as the labeling reagent (i.e. the compound or moiety prior to the binding with the peptide or protein). A label used in the present invention may be attached to an amine group, carboxyl group or an amino acid residue of a protein, polypeptide and/or peptide via a covalent or a non-covalent linkage. Typically, the linkage is a covalent linkage. The labels can be selected *inter alia* from isotopic labels, isobaric labels, enzyme labels, colored labels, fluorescent labels, chromogenic labels, luminescent labels, radioactive labels, haptens, biotin, metal complexes, metals, and colloidal gold, with isotonic labels and isobaric labels being particularly preferred. All these types of labels are well established in the art.

The labeling steps (a) and (b) may be a single chemical labeling or a double chemical labeling step.

The term "single chemical labeling", as used herein, denotes that a protein and/or peptide is labeled with one or more detectable markers of only one type of labels, for example, only isobaric labels. The term "double chemical labeling", as used herein, denotes that a protein and/or peptide is labeled with one or more detectable markers of two different types of labels, for example, isotopic labels and isobaric labels.

The term "isobaric labels" as used herein refers to a group of at least two labels which have the same mass but which upon collision induced dissociation (CID) may generate fragments of different masses. To this end an isobaric label consists of a reporter group of varying mass and a balance group which compensates for the different mass of the various reporter groups that are generated upon CID. In a preferred embodiment, isobaric labels will not only have the same mass before they are fragmented by CID, but also the same structure. The differential mass distribution between a reporter and a balance group may be achieved by differential distribution of isotopes within the isobaric labels. A schematic example for such isobaric labels is depicted in Figure 3.

Examples of isobaric labels according to the invention include *inter alia* Isobaric Tag for Relative and Absolute Quantitation (iTRAQ) labels (cf. Ross, P.L. et al. (2004) Mol. Cell. Proteomics 3, 1154-1169). This approach employs four different iTRAQ reagents, each containing a reporter group, a balance group and a peptide reactive group which reacts with primary amine groups. The reporter group has a mass of 114, 115, 116 or 117 Da, depending on differential isotopic combinations of ¹²C/¹³C and ¹⁶O/¹⁸O in each reagent. The balance group varies in mass from 31 to 28 Da to ensure that the combined mass of the reporter group and the balance group remains constant (145 Da) for the four reagents. Accordingly, labeling of e.g. the same peptide with each of these reagents results in peptides which are isobaric and thus co-elute, for example, in liquid chromatography and consequently are chromatographically indistinguishable from each other. During mass spectrometry, however, at least the respective reporter groups are released upon CID, displaying distinct masses of 114 to 117 Da. The intensity of these fragments can be used for quantification of the individual proteins and/or peptides in a single.

The advantage of using isobaric labels is that one can label identical proteins, polypeptides or peptides of different samples with different isobaric labels and analyze them in a mass spectrometry analysis. Identical proteins, polypeptides or peptides from different samples which have been labeled with such isobaric labels as well as fragments resulting thereof will yield the same signal in a mass spectrometry analysis. However, upon CID the signal of masses will be split up into different signals, each signal being characterized by a different mass resulting from the different reporter group mass allowing for the analysis of the relative distribution of these identical proteins, polypeptides or peptides within the different samples originally used.

The term "isotopic label" refers to a set of at least two or more labels having the same chemical formula but differing from each other in the number and/or type of isotopes present for one or more atoms. The advantage of using isotopic labels is that one can label identical proteins, polypeptides or peptides of different samples with different isotopic labels and analyze them in a mass spectrometry analysis. Identical proteins, polypeptides or peptides from different samples which have been labeled with such isotopic labels as well as fragments resulting thereof will yield the signal in a mass spectrometry analysis that differ exactly by the weight difference of the isotopic labels which is due to the use of the different isotopes. In other words, otherwise identical proteins, polypeptides and/or peptides from different samples labeled with different isotopic labels can be differentiated in a mass spectrometry analysis as such, based on different mass.

Examples of isotopic labels according to the invention include *inter alia* ¹⁶O- or ¹⁸O-labeled water or Isotope-Coded Affinity Tag (ICAT) labels (cf. Gygi, S.P. et al. (1999) Nat. Biotechnol. 17, 994-999). The ICAT reagent uses three functional elements: a thiol-reactive group for the selective labeling of reduced cysteine amino acid residues, a biotin affinity tag to allow for selective isolation of labeled peptides, and an isotopic tag, which is synthesized in two isotopic forms, the "light" (non-isotopic) and the "heavy" (utilizing, for example, ²H or ¹³C) form. Within the scope of the present invention, isotopic labeling may be performed on the peptide level (e.g., by cleaving the proteins comprised in the sample to be analyzed in the presence of either ¹⁶O- or ¹⁸O-labeled water) or directly on the protein level (i.e. prior to cleavage), for example by employing commercially available ICAT reagents (Applied Biosystems, Foster City, CA, USA).

While isobaric labels (see above) in principle constitute a specific type of isotopic labels, in the context of the present invention, the term "isotopic labels" will be used to refer to labels which are not isobaric, but can as such be differentiated in a mass spectrometry analysis based on their molecular weight before CID is performed.

For the purposes of the present invention, labels and preferably the isotopic and/or isobaric labels may further comprise a reactive group which allows their coupling to proteins, polypeptides and/or peptides. The reactive group will preferably allow covalent linkage between the isotopic and/or isobaric labels and the proteins, polypeptides and/or peptides. The reactive groups may be selected from the group in coupling chemistries comprising he microcarrier beads, beads may be used that provide functional groups such as carboxyl groups, amine groups, hydroxyl groups, sulfhydryl groups etc. Other covalent attachment chemistries are also applicable as reactive group and include without being limited thereto anhydrides, epoxides, aldehydes, hydrazides, acyl azides, aryl azides, diazo compounds, benzophenones, carbodiimides, imidoesters, isothiocyanates, NHS esters, CNBr, maleimides, tosylates, tresyl chloride, maleic acid anhydrides and carbonyldiimidazole. NHS esters are preferred as coupling groups.

Preferably, these coupling chemistries are selected to allow the coupling of labels to primary amines within proteins, polypeptides and/or peptides, i.e. to the amino group at the N-terminus of proteins, polypeptides and/or peptides.

Thus, a coupling chemistry may preferably be NHS esters if one attempts to label proteins, polypeptides and peptides at their available amine groups. However, if the target group within the proteins, polypeptdides and/or peptides is sulfhydryl group from cystein, one may use a thiol reactive coupling chemistry such as iodoacetamide.

The labels and preferably the isobaric and/or isotopic labels may further comprise a so called affinity group. Such affinity groups as used herein allow the isolation of labeled fragments of proteins, polypeptides and/or peptides by certain chromatographic approaches. To this end, one may use affinity groups as they are known in the art to selectively enrich proteins, polypeptides and/or peptides. These include affinity tags such as the glutathion-S-transferase tag, the poly-Histidine-tag, the biotine-tag, the HA-tag, the Flag-tag etc. Proteins, polypeptides and/or peptides which have been labeled with markers comprising such affinity groups may be isolated using chromatographic media such as glutathione-S-transferase sepharose, immobilized metal affinity chromatography resins such as Nickel-NTA sepharose, streptavidine-sepharose, anti-HA-antibody based chromatography, anti-FLAG-antibody based chromatography etc.

The affinity and/or reactive groups may be differentially arranged within labels and preferably within the isobaric and/or isotopic labels. The non-exclusive arrangement of these groups within isobaric and/or isotopic labels is depicted in the following formulas.

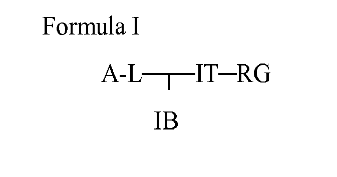

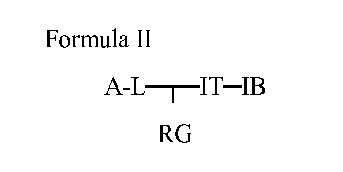

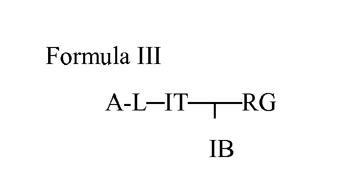

Formula IV A-L-IB-IT-RG

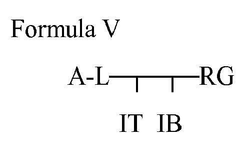

wherein A specifies at least one affinity group for the reversible, covalent or non-covalent binding to an affinity matrix; IB is a group comprising an isobaric tag group; IT is a group comprising an isotopic tag group; L specifies a linker containing a cleavable moiety; and RG is a reactive group for the selective binding of biomolecules such as, but not limited to proteins or peptides. In a preferred embodiment of the invention the isobaric group is located at the terminal end of the inventive molecules as shown in formula I, II, III and V. A particularly preferred embodiment relates to labels of formula V.

The skilled person will be aware that it may be preferred to make the affinity group as small as possible to allow better coupling efficacy etc. One may also consider to incorporate the affinity group within the arrangement of atoms forming the isotopic part of the label. For example, one may use histidine or leucine residues with a different distribution of isotopes. In such cases, the affinity group and the isotopic group become identical and the size of the label can be further reduced. In case of histidine the affinity tag may be recognized by IMAC (immobilized metal affinity chromatography) resins such Ni-NTA-Sepharose. In case of poly-Leu-based tags, one may use coomercially available anti-Leucine antibodies.

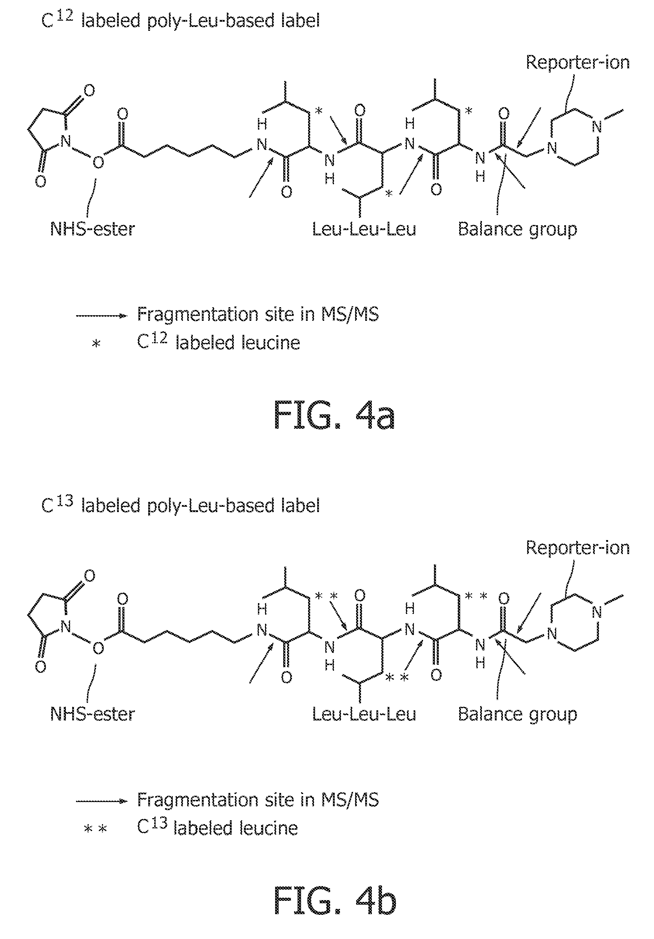

Thus, in a preferred embodiment, isotopic labels and e.g. the affinity group may be combined. For example one may use a poly-His-tag or poly-Leu-tag in which the single histidine or leucine amino acids are labeled differently with ¹²C or ¹³C and/or ¹⁴N and ¹⁵N. A representative example of such a preferred embodiment is depicted in Figure 4. The labels depicted in Fig. 4 may be particularly preferred in the context of the invention.

Thus a particularly preferred embodiment in general uses labels of formula VI: 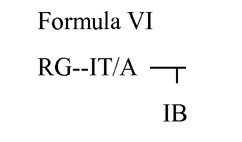 wherein RG, IT, A and IB are as defined above and wherein IT and A are formed by the same molecular entity.

The present invention in particularly preferred embodiments relates to the combined use of isotopic labels and isobaric labels for multiplexed protein analysis, that is for performing multiple analyses in parallel, for example 2, 4, 8 or 16 parallel samples. In particular, such a combined labeling strategy also enables the comparison of relative protein levels between different samples. The combination of isotopic and isobaric labeling may have the advantage that only those peptides need to be specifically analyzed, for example by MALDI-MS/MS analysis or iTRAQ quantification, for which a differential expression level is observed, thus resulting in a faster and less complex sample analysis.

For example one may label four samples taken from patients suffering from a disease with a first set of labels which comprise an identical isotopic label but four different isobaric label. The isobaric labels may be chosen to have the same structure but to yield four different reporter groups upon CID. One may use the iTRAQ labels described above. One then can label four samples from a control population with a second set of labels which comprise again the same isotopic label but different isobaric labels. The isobaric label may be exactly the same as for the first four samples. However, the isotopic label may differ from the isotopic label for the first four samples by e.g. single isotope ¹²C/¹³C exchange. By first detecting the isotopic label through mass differences one can identify those proteins which e.g. are overexpressed in patients vs the control group. The peaks identified this way can then be analyzed in a MS/MS scan. After CID the relative contribution of the four samples in the patint group to e.g. a single peak can be analyzed by detecting the isobaric labels.

The skilled person will understand that once the proteins, polypeptides and/or peptides have been labeled, one will have to block remaining available amine groups such as those being present e.g. in lysine and arginine before the proteins, polypeptides and/or peptides are digested.

Thus, in one preferred embodiment one may use labels which have a coupling chemistry that allows their coupling to amine groups within proteins, polypeptides and/or peptides. If these labels are used in excess, they allow to modify the N-terminal amine group of proteins, polypeptides and/or peptides as well as the amine group found within amino acids such as lysine or arginine. In such cases, all amine groups within proteins, polypeptides and/or peptides are modified and no further blockage is necessary. In an alternative, one may use such label not in excess and then block the remaining amine groups which have not been modified with the labels before the proteins, polypeptides and/or peptides are digested. The labels may preferably be isotopic labels which may optionally comprise an isobaric label.

In another preferred embodiment one may use labels which have a coupling chemistry that allows their coupling to groups other than amine groups within proteins, polypeptides and/or peptides. Such groups may be the sulfhydryl group of e.g. cysteine. In such cases, all amine groups within proteins, polypeptides and/or peptides will have to be blocked before the proteins, polypeptides and/or peptides are digested. The labels may preferably be isotopic labels which may optionally comprise an isobaric label.

One may block free amine groups still present within proteins, polypeptides and/or peptides after labeling by using acetylation with for example sulfo-*N-*hydroxysuccinimide acetate.

Thus, the present invention in one aspect requires that any free amine groups present within proteins, polypeptides and/or peptides are modified before digestion so that they can not be methylated later on.

The term "digesting the labeled proteins, polypeptides and/or peptides to generate fragments thereof" as used herein refers to the chemical and/or enzymatic cleavage to generate fragments of the labeled proteins, polypeptides and/or peptides.

Such cleavage of e.g. proteins may either be achieved chemically, e.g., via acid or base treatment employing chemicals such as cyanogen bromide, 2-(2'-nitrophenylsulfonyl)-3-methyl-3-bromo-indolenine (BNPS), formic acid, hydroxylamine, iodobenzoic acid, and 2-nitro-5-thiocyanobenzoid acid) or enzymatically via proteases including *inter alia* trypsin, pepsin, thrombin, papain, and proteinase K all of which are well known in the art.

The terms "fractionation" and "re-fractionation", as used herein, refer to any type separation process in which the proteins, polypeptides and/or peptides as well as the fragments thereof as they are present in a sample in a certain quantity are divided (i.e. sorted) up in a large number of smaller quantities (i.e. fractions) according to any differences in their physico-chemical properties such as the molecular mass, their size, and their overall net charge. A common trait in fractionations is the need to find an optimum between the amount of fractions collected and the desired purity in each fraction. Fractionation makes it possible to isolate more than two components in a mixture in a single run. This property sets it apart from other separation techniques. There are several methods for fractionating proteins and/or peptides well established in the art including classical SDS polyacrylamide gel electrophoresis (SDS-PAGE), two-dimensional gel electrophoresis, size-exclusion chromatography, (two-dimensional) liquid chromatography, and isoelectric focusing, with the latter one being particularly preferred. Methods for analysis, particularly visualization, of the proteinaceous molecules after fractionation has taken place are well established in the art.

Isoelectric focusing is a technique for separating different molecules by their electric charge differences. It is a type of zone electrophoresis, usually performed in a gel (e.g., an agarose gel or, preferably, polyacrylamide gel) or in liquid phase, that takes advantage of the fact that a molecule's charge changes with the pH of its surroundings. The molecules to be focused are distributed over a medium that has a pH gradient. An electric current is passed through the medium, creating a "positive" anode and "negative" cathode end. Negatively charged molecules migrate through the pH gradient in the medium toward the "positive" end while positively charged molecules move toward the "negative" end. As a particle moves towards the pole opposite of its charge it moves through the changing pH gradient until it reaches a point in which the pH of that molecules isoelectric point (pI) is reached. At this point the molecule no longer has a net electric charge (due to the protonation or deprotonation of the associated functional groups) and as such will not proceed any further within the gel. Isoelectric focusing can resolve proteins that differ in pI value by as little as 0.01.

Isoelectric focusing is usually the first step in two-dimensional gel electrophoresis, in which proteins are first separated by their pI and then further separated by molecular weight through standard SDS-PAGE.

If labels in accordance with the invention comprise affinity groups or tags, one can also use the respective affinity resin that is specific for the affinity group for fractionation purposes. For example, one can use streptavidine based resins for fractionation of biotine-labeled fragments.

The term "re-fractionating", as used herein, also denotes that the method of the invention may be performed with a single type of fractionation method, that is, the proteins and/or peptides present in a sample are fractionated before and after performing a chemical reaction such as methylation and/or removing and/or altering a specific post-translational modification, particularly a phosphate group, by applying the same method, preferably isoelectric focusing. However, depending on the particular protocol it may also be possible to use different methods, e.g. isoelectric focusing and classical SDS-PAGE, affinity fractionation etc..

Other methods of fractionation/re-fractionation and combinations thereof may also be envisaged. Thus, fractionation/re-fractionation may rely on ion exchange chromatography, size exclusion chromatography, hydrophobic interaction chromatography, reversed-phase chromatography and/or (immuno)affinity chromatography.

The term "methylation reaction" as used herein, refers to the methylation of primary amine groups by chemical or enzymatic reaction. The person skilled in the art is well aware how to perform such a methylation reaction. Typically, to this end, one may use e.g. 2,4,6-trinitrobenzenesulfonic acid (TNBS).

In short, the dried digested fragments are redissolved in 50 µl of 50 mM sodium borate (pH 9.5). Then, 2 µmol of dried TNBS is redissolved in 200 µl of 50 mM sodium borate (pH 9.5), of which 15 µl is transferred to each sample. The peptides are incubated with TNBS for 55 min at 37 °C. This reaction is repeated once, after which the samples are dried. The modification procedure, including the two reaction steps, is repeated once to assure substantially quantitative TNBS modification.

Free amines could also be acetylated in 10 mM sulfo-*N-*hydroxysuccinimide acetate for 90 min at 30 °C. Partial acetylation of serine and threonine could be reversed by adding 1 µl of hydroxylamine to the protein mixture.

The methylating agents and reaction conditions will be chosen to allow for methylation of primary amine groups. Thus, the methylation agents and conditions of the methylation reaction may be chosen to allow for labeling of N-terminal NH₂ groups that are generated upon chemical or enzymatic digestion of the labeled proteins, polypeptides and/or peptides. Methylation of amine groups of e.g. the amino acids lysine and arginine is prevented by the prior blockage of these groups or the complete modification of these groups with the labels (see above).

Using the methods in accordance with the present invention, it is possible to reduce the complexity of mass spectrometry analysis by looking only at N-terminal fragments that are representative of the proteins, polypeptides and/or peptides within samples. Thus, the labeling step is performed only to label the primary amines found at the N-terminus of proteins, polypeptide and/peptides. After digestion of these labeled species, new primary amines are generated at the respective N-termini of the resulting fragments. The subsequent methylation reaction changes the physico-chemical behaviour of these newly generated fragments so that they should behave differently in chromatographic separations before and after methylation. If the labeled fragments are thus first subjected to a fractionation step, then methylated and then again subjected to the same chromatographic separation principle, only those fractions should differ with respect to the chromatographic behaviour for which the methylation reaction has been successful. Those fractions, however, which remain unchanged should be the N-terminal peptides which have been labeled upfront before the proteins, polypeptides and/or peptides were digested.

The methods in accordance with the invention thus allow for a significant reduction of the complexity of signals to be analyzed whilst the qualitative and quantitative outcome of the analysis, in principle, should not change because representative fragments of each protein, polypeptide and/or peptide are analysed.

Another advantage of using the methods of the present invention is that it allows for the analysis of differentially spliced proteins. For example, it is estimated that 30 to 50% of all transcripts lead to spliced mRNA's generating different protein isoforms which to some degree differ with respect to their N-terminus. Thus, it is known that many protein isoforms are expressed in a tissue-specific manner, i.e. different splice variants are selectively expressed in dedicated cell types but not in others. Differential expression of protein isoforms under disease conditions could therefore derive atissue-specific disease marker. However, with the standard mass spectrometry approaches it is difficult to monitor ion formation on different spliced variants of certain protein as the variant-specific information is often only contained in a few tryptic peptides at the N-terminal and of the protein.

As the present invention allows for specific selection of N-terminal peptides for mass spectrometric analysis, it may be possible to identify and characterize disease-specific splice activities.

It is further well known that proteins, polypeptides and/or peptides may be post-translationally modified such as with phosphate groups or with e.g. glycosyl moieties. As differential post-translational modification can be implicated in e.g. disease development, there is an interest in identifying such proteins in which the differential post-translational modification occurs. Therefore, the present invention in one embodiment relates to:
A method for the selective enrichment and/or separation of N-terminal fragments of polypeptides and/or peptides in a sample, comprising:
   (a) labeling of proteins, polypeptides and/or peptides comprised within at least a first sample with at least one first label;
   (b) labeling of proteins, polypeptides and/or peptides comprised within at least a second sample with at least one second label;
   (c) combining the labeled proteins, polypeptides and/or peptides of step (a) and (b);
   (d) digesting the labeled proteins, polypeptides and/or peptides to generate fragments thereof;
   (e) fractionating said fragments;
   (f) performing a methylation reaction with said fragments;
   (g) re-fractionating said fragments;
   (h) comparing the fractionation patterns obtained in steps (e) and (g); and
   (i) separating N-terminal fragments of the polypeptides and/or peptides of steps (a) and (b) which carry a label based on the results obtained in step (g) fractionating the isolated N-terminal fragments;
   (j) fractionating the isolated N-terminal fragments;
   (k) removing or altering the phosphate-group from at least a first subset of N-terminal fragments identified in step (i);
   (l) re-fractionating the isolated N-terminal fragments;
   (m) comparing the fractionation patterns obtained in steps (j) and (l); and
   (n) separating the at least first subset of phospho-N-terminal fragments modified in step (k) based on the results obtained in step (m).

In particular, the present invention thus relates to the selective enrichment and/or separation of phospho-proteins, phospho-polypeptides and/or phospho-peptides which have a phosphate group in an N-terminal fragment.

The term "removing", as used herein, refers to the complete elimination of a phosphate-group, for example by chemical cleavage or enzyme action (see also the discussion below). The term "altering", as used herein, denotes any modification of the phosphate-group resulting in a change in the physico-chemical properties of the proteins, polypeptides and/or peptides that allows a discrimination between the variants bearing the original and the altered post-translational phosphate-group. Examples of such alterations include *inter alia* the cleavage of one or more chemical bonds within the phosphate group without removing it from the amino acid backbone as well as the conjugation of the phosphate group with any moiety, thus resulting in an increase in molecular weight. Typically, the treatment of the proteins, polypeptides or peptides as well as fragments thereof is performed under reaction conditions ensuring the removal and/or alteration of all post-translational phosphate-groups that are comprised in the molecules in order to avoid the occurrence of any "intermediate molecules" where one or more phosphate-groups have been removed and/or altered but one or more others remain unchanged.

The removal and/or alteration of the post-translational phosphate modification results in an alteration of the physico-chemical properties of only the at least first subset of phosphorylated N-terminal fragments derived from phopho-proteins, - polypeptides and/or -peptides that have a phosphate group modification in an N-terminal fragment compared to the remaining N-terminal fragments of proteins, polypeptides and/or peptides present in the sample such that this at least first subset can be identified during the subsequent re-fractionation step, since only this at least first subset will sort into a different fraction. All other N-terminal fragments which did not carry a phosphate modification will sort into the same fraction as during the initial fractionation step prior to removing/altering the phosphate-group from the at least first subset of N-terminal fragments.

Thus, for identifying and/or separating the at least first subset N-terminal of phospho-proteins, -polypeptides and/or -peptides the results obtained in the initial and the re-fractionation step have to be compared (for example, comparing the specific patterns obtained after staining the gel used for electrophoretic protein separation).

In order to facilitate the sorting/separation process the fractions obtained after the initial fractionation step are preferably treated individually to removing/altering the post-translational phosphate-group before applying them to re-fractionation.

In further preferred embodiments of the invention, the method is used for the discrimination of different subsets of phosphorylated N-terminal fragments, namely serine/threonine-phosphorylated (i.e. the phosphate is attached to a serine or a threonine amino acid residue) and tyrosine-phosphorylated N-terminal fragments (i.e. the phosphate is attached to a tyrosine amino acid residue). Since these subsets of phospho-proteins and/or -peptides are regulated by different kinases and phosphatases, they may have also been implicated in different cellular processes or signaling cascades.

Importantly, the two above subsets of N-terminal fragments derived from phospho-proteins, -polypeptides and/or -peptides also differ in their accessibility to chemical treatment with regard to the removal and/or alteration of post-translational modifications. For example, serine- and threonine-phosphorylated proteinaceous molecules are accessible to the chemical removal of the phosphate group via β-elimination (i.e., a type of elimination reaction well established in the art, wherein atoms or atom groups are removed from two adjacent atoms of the substrate while forming a π bond), whereas tryrosine-phosphorylated proteinaceous molecules are not. This difference provides a basis for distinguishing these two subsets of N-terminal fragments of phospho-proteins and/or -peptides.

Thus, in preferred embodiments of the invention, the method is configured to allow the discrimination between serine- and threonine-phosphorylated and tyrosine-phosphorylated N-terminal fragments of phosphorylated proteins, polypeptides and/or peptides, respectively. Based on the above-mentioned accessibility to chemical treatment it is particularly preferred that the at least first subset of N-terminal fragments of phospho-proteins, -polypeptides and/or -peptides comprises serine- and threonine-phosphorylated N-terminal fragments. In other words, following the initial fractionation step the first subset to be enriched and/or separated from tyrosine-phosphorylated and unphosphorylated N-terminal fragments comprises serine- and threonine-phosphorylated N-terminal fragments.

Preferably, in any embodiments of the invention relating to the analysis of N-terminal fragments of phospho-proteins,-polypeptides and/or -peptides the phosphate-group is removed chemically via β-elimination. However, it is also possible to remove the phosphate-group, for example, enzymatically by means of specific or non-specific phosphatases.

To specifically remove the phosphate moieties from phospho-serine and phospho-threonine amino acid residues, β-elimination is typically induced by base hydrolysis. In order to avoid any adverse effects on the side chains of cysteine and methionine residues, the samples may first be treated with performic acid, resulting in the oxidation, and thus inactivation of these residues. A typical reaction mixture for performing β-elimination comprises H₂O, dimethyl sulfoxide, acetonitrile, 250 mM EDTA (pH 8.0), and 5 M NaOH.. The samples are incubated for 1 h at 55°C under a N₂ atmosphere, cooled to room temperature, and quenched by neutralizing with acetic acid. Removing the phosphate-group via β-elimination results in forming an unnatural amino acid (e.g., dehydro-alanine, dehydro-2-amino butyric acid).

After subjecting the individual fractions obtained after the initial fractionation step to the above treatment, the are re-fractionated, typically by applying the same fractionation method, preferably isoelectric focusing.

The removal and/or alteration of the phosphate group specifically from phospho-serine and phospho-threonine amino acid residues results in an alteration of the physico-chemical properties of only those subset of N-terminal fragments (i.e. the at least first subset) comprising such modified amino acid residues such that they can be identified during the subsequent re-fractionation step, since only this subset will sort into a different fraction. All other proteins and/or peptides comprised in the sample includingN-terminal fragments of tyrosine-phosphorylated and - of course - unphosphorylated proteins, -polypeptides and/or -peptides will sort into the same fraction as during the initial fractionation step prior to removing/altering the post-translational phosphate-group. Thus, using the above-described method, N-terminal fragments of serine- and threonine-phosphorylated-proteins, -polypeptides and/or -peptides can be selectively enriched from a complex sample by comparing the results of the initial and the re-fractionation step performed.

In another embodiment, the inventive method further comprises the selective enrichment and/or separation of at least a second subset of N-terminal fragments of phospho-proteins, -polypeptides and/or -peptides from the subset of proteins and/or peptides remaining after separation of the at least first subset following the re-fractionation step, as described above. To accomplish this goal, the remaining subset of N-terminal fragments is subjected to another cycle of fractionation, removing or altering the post-translational phosphate modification, and re-fractionation, wherein the phosphate-group from at least a second subset of N-terminal fragments of phospho-proteins and/or -peptides is removed or altered.

Particularly preferably, the at least second subset of N-terminal fragments of proteinaceous molecules are of tyrosine-phosphorylated proteinaceous molecules. Since phosphorylated tyrosine residues are not accessible to chemical cleavage via β-elimination, it is preferred to remove the phosphate group enzymatically. Enzymatic removal may be achieved via phosphatases, particularly via alkaline phosphatases (for the non-specific removal of phosphate-groups) or via protein tyrosine phosphatases (for specifically dephosphorylating phospho-tyrosine residues). Such phosphatases are commercially available.

By performing a second re-fractionation step the N-terminal fragments of tyrosine-phosphorylated proteins, polypeptides and/or peptides can be separated from their unphosphorylated counterparts based on the same principle as described above. The remaining subset of proteins and/or peptides may optionally be subjected to a third, forth, and so forth cycle of fractionation, removing/altering a post-translational modification, and re-fractionation, wherein the type of post-translational modification to be removed or altered is typically not phosphorylation but, for example, ubiquitinylation or glycosylation.

Additionally and/or alternatively, the present invention relates to the selective enrichment and/or separation of N-terminal fragments resulting from glycosylated proteins, polypeptides and/or peptides. The identification of such fragments may follow the same lines as has been described above for e.g. phospho-proteins. Thus, the present invention in a further embodiment relates to:
A method for the selective enrichment and/or separation of N-terminal fragments of polypeptides and/or peptides in a sample, comprising:
   (a) labeling of proteins, polypeptides and/or peptides comprised within at least a first sample with at least one first label;
   (b) labeling of proteins, polypeptides and/or peptides comprised within at least a second sample with at least one second label;
   (c) combining the labeled proteins, polypeptides and/or peptides of step (a) and (b);
   (d) digesting the labeled proteins, polypeptides and/or peptides to generate fragments thereof;
   (e) fractionating said fragments;
   (f) performing a methylation reaction with said fragments;
   (g) re-fractionating said fragments;
   (h) comparing the fractionation patterns obtained in steps (e) and (g); and
   (i) separating N-terminal fragments of the polypeptides and/or peptides of steps (a) and (b) which carry a label based on the results obtained in step (g) fractionating the isolated N-terminal fragments;
   (j) capturing a subset of glycosylated N-terminal fragments; and
   (k) separating the captured subset of glycosylated N-terminal fragments.
Typically, the capturing step comprises at least one affinity purification or affinity chromatography step, that is, the attachment (i.e. capturing) of the subset of glycosylated N-terminal fragments to a binding member having specific binding activity for the subset of N-terminal fragments to be selected. However, the capturing step may also may rely on one or more of ion exchange chromatography, size exclusion chromatography, hydrophobic interaction chromatography and/or reversed-phase chromatography. Within the scope of the present invention, it is also possible to combine two or more capturing steps of the same or of different types, for example two affinity chromatography steps (either using the same type of matrix or different types) or an affinity purification step and a ion exchange chromatography.

In preferred embodiments relating to the analysis of glycosylated N-terminal fragments the capturing step comprises at least one of lectin affinity capture and glycoprotein chemical capture. The term "lectin affinity capture", as used herein, denotes any capturing protocol employing lectins as a binding member. The term "lectins", as used herein, refers to a class of proteins found in plants, bacteria, fungi, and animals that are known to bind specific oligosaccharide moieties (reviewed, e.g., in Lis, H., and Sharon, N. (1998) Chem. Rev. 98, 637-674). Unlike antigen-antibody binding affinities, the affinity constants for the binding of monosaccharides and oligosaccharides to most lectins are in the low micromolar range but can be in the millimolar range. For affinity capture purposes, it is the multivalent nature of both the oligosaccharides and the lectins themselves that make these interactions useful for chromatography separations. Examples of suitable lectins include *inter alia* α-sarcin, rizin, concavalin A, and calnexin. Several protocols for lectin affinity capture are known in the art (cf, e.g., Kaji, H. et al. (2003) Nat. Biotechnol. 21, 667-672; Hirabayashi, J. (2004) Glycoconj. J. 21, 35-40; Drake, R.R. et al. (2006) Mol. Cell. Proteomics 5, 1957-1967).

The term "glycoprotein chemical capture", as used herein, refer sto any chemical capture procedures for glycoproteins not involving the use of lectins. Many of these procedures involve an ion exchange chromatography step. Several protocols are well established in the art (cf., e.g., Zhang, H. et al. (2003) Nat. Biotechnol. 21, 660-666; Sun, B. et al. (2007) Mol. Cell. Proteomics 6, 141-149).

The present invention also relates to:
A method for the selective enrichment and/or separation of N-terminal fragments of polypeptides and/or peptides in a sample, comprising:
   (a) labeling of proteins, polypeptides and/or peptides comprised within at least a first sample with at least one first label;
   (b) labeling of proteins, polypeptides and/or peptides comprised within at least a second sample with at least one second label;
   (c) combining the labeled proteins, polypeptides and/or peptides of step (a) and (b);
   (d) digesting the labeled proteins, polypeptides and/or peptides to generate fragments thereof;
   (e) fractionating said fragments;
   (f) performing a methylation reaction with said fragments;
   (g) re-fractionating said fragments;
   (h) comparing the fractionation patterns obtained in steps (e) and (g); and
   (i) separating N-terminal fragments of the polypeptides and/or peptides of steps (a) and (b) which carry a label based on the results obtained in step (g) fractionating the isolated N-terminal fragments;
   (j) fractionating the isolated N-terminal fragments;
   (k) removing or altering the glycosyl-group from at least a first subset of N-terminal fragments identified in step (j);
   (l) re-fractionating the isolated N-terminal fragments;
   (m) comparing the fractionation patterns obtained in steps (j) and (l); and
   (n) separating the at least first subset of glycosylated N-terminal fragments modified in step (k) based on the results obtained in step (m).

Preferably, the glycosyl group is removed chemically (for example, via β-elimination) or enzymatically by means of particular glycosidases.

In one embodiment of the inventive method relating to the selective enrichment and/or separation of glycosylated N-terminal fragments of proteins, - polypeptides and/or -peptides the at least first subset of the separated glycosylated N-terminal fragments comprises *N*-glycosylated N-terminal fragments.

Removal of an *N*-linked gycosyl modification from the N-terminal fragments may be accomplished by enzymatic cleavage of the *N*-linked peptides from the glycosyl moiety at 37 °C overnight using peptide:*N*-glycosidase F (PNGase F) at a concentration of 500 U (1 µl) PNGase F per 2-6 mg of crude proteins. PNGase F is an amidase that cleaves between the innermost GlcNAc and asparagine residues of high mannose, hybrid, and complex oligosaccharides from *N*-linked glycol-proteins. The supernatant containing the released de-glycosylated peptides can be collected by centrifugation. Thus, this procedure allows for the selective discrimination of *N-*glycosylated proteins and/or peptides from the other types of glyco-proteins and/or - peptides (i.e. *O*-glycosylated and GPI-anchored proteins and/or peptides, respectively).

Although PNGase F deglycosylation removes the sugar moiety from the glyco-peptide, the glycosylation site can still be detected by mass spectrometry analysis, since PNGase F deglycosylation results in an aspartic acid for every asparagines. (corresponding to a mass difference of + 1 Da).

In another typical embodiment, the inventive method, particularly the separation step, further comprises removing the post-translational modification from at least a second subset of the separated glycosylated N-terminal fragments. In one embodiment of the inventive method relating to the selective enrichment and/or separation of glycosylated N-terminal fragments, the at least second subset of the separated glycosylated N-terminal fragments comprises *O*-glycosylated N-terminal fragments.

Removal of an *O*-linked gycosyl modification from these fragments may be accomplished by enzymatic cleavage employing particular *O*-glycosidases or chemically such as via a β-elimination (i.e., a type of elimination reaction well established in the art, wherein atoms or atom groups are removed from two adjacent atoms of the substrate while forming a π bond).

Other combinations of the above-mentioned methods will be apparent to the skilled person.

In other embodiments, the above methods further comprises analyzing the isolated N-terminal fragments (which may or may not be e.g. phosphorylated and/or glycosylated N-terminal fragments) by means of mass spectrometry, an analytical technique used to measure the mass-to-charge ratio of ions. Importantly, for phospho-N-terminal fragments mass spectrometric analysis is facilitated by the removal of the unstable phosphate-group but leaving behind a unique unnatural amino acid for the serine and threonine-phosphorylated species. The particular mass spectrometric analysis applied may depend on the levels of protein and/or peptide expression determined in different samples. In some embodiments, the method of the invention are performed in a high-throughput format.

In a further embodiment, the invention relates to the use of a method, as described herein, for performing qualitative and/or quantitative proteomic analyses.

The combined use of isotopic and isobaric labels as mentioned above allows for performing multiple analysis in parallel, for example, 2, 4, 8 or 16 parallel samples. The ability to perform multiple mass spectrometry analysis combined with a reduced complexity of the analysis in view of the selective enrichment of N-terminal fragments may be a powerful tool in the identification of disease markers. For example, one may take samples from 4 different individuals that are known not to suffer from specific disease and label these molecules with a combined isotopic and isobaric label as mentioned above. If one uses for example an iTRAQ label as an isobaric label that can generate up to 4 reporter groups of different mass, it is possible to label all proteins from these 4 individuals.

In parallel, one may take samples from 4 individuals known to suffer from a specific disease and use the combined isotopic isobaric label wherein the isotopic label differs from the isotopic label that has been used to label the samples of the healthy individuals while the isobaric label is the same. If one now combines these labeled samples digests them and performs the method of the invention as described, one will obtain the N-terminal fragments of four samples of the healthy individuals and four samples of patients. Differentially expressed peptides between patients and healthy individuals will be recognizable in a mass spectrometry analysis as by the change in mass due to the different isotopic labels. If a subsequent analysis a MS/MS-scan is performed, one can analyze such identified mass peaks with respect to the individual peak distribution for the four healthy individuals and for the four patients.

Thus, one can largely increase the throughput of such analysis and at the same time increase the signal to noise ratio given that all 8 samples as regards the isolation of the N-terminal fragments have been treated identically. The source of error is thus significantly reduced. The present invention therefore also relates to the use of a method as described herein for performing quantitative and qualitative proteomic analysis.

Whilst the above invention has been described with respect to some of its preferred embodiments, these embodiments are in no way mentioned to limit the scope of the invention. The person skilled in the art is clearly aware of further embodiments and alterations to the previously described embodiments that are still within the scope of the present invention.

### EXAMPLES

### Example 1 - Methylation of N-terminal fragments

2,4,6-trinitrobenzenesulfonic acid (TNBS) is used to methylate the free amino groups of tryptic fragments resulting from proteins, polypeptides and/or peptides which have been labeled with e.g. isotopic label that optionally comprise isobaric lables. In short, the dried peptides are redissolved in 50 µl of 50 mM sodium borate (pH 9.5). Then, 2 µmol of dried TNBS is redissolved in 200 µl of 50 mM sodium borate (pH 9.5), of which 15 µl is transferred to each sample. The peptides are incubated with TNBS for 55 min at 37 °C. This reaction is repeated once, after which the samples are dried. The modification procedure, including the two reaction steps, is repeated once to assure substantially quantitative TNBS modification.

Free amines can also be acetylated in 10 mM sulfo-*N*-hydroxysuccinimide acetate for 90 min at 30 °C. Partial acetylation of serine and threonine can be reversed by adding 1 µl of hydroxylamine to the protein mixture.

### Example 2 - Synthesis of a (Leu)₃ labeling compound

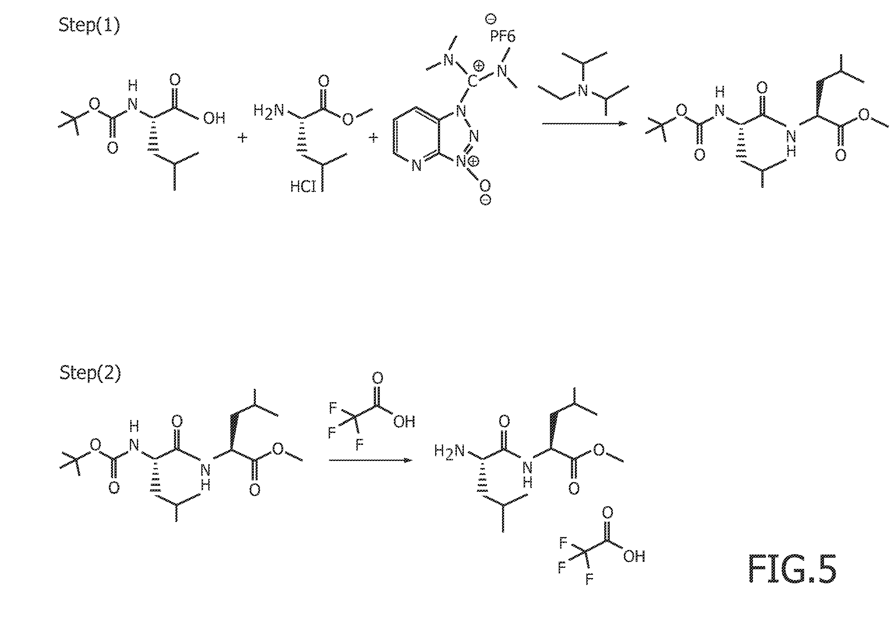

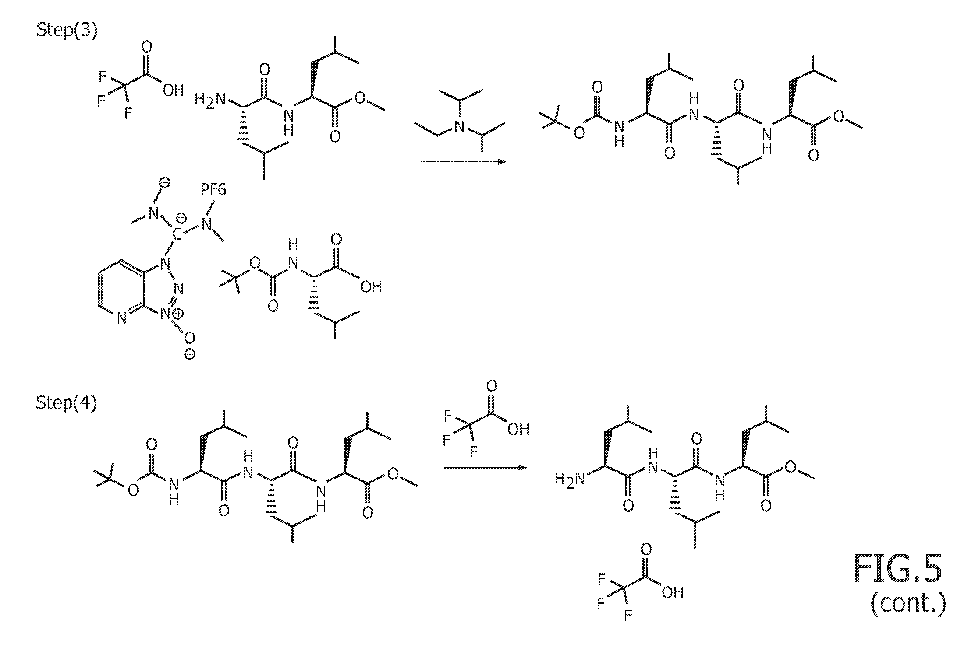

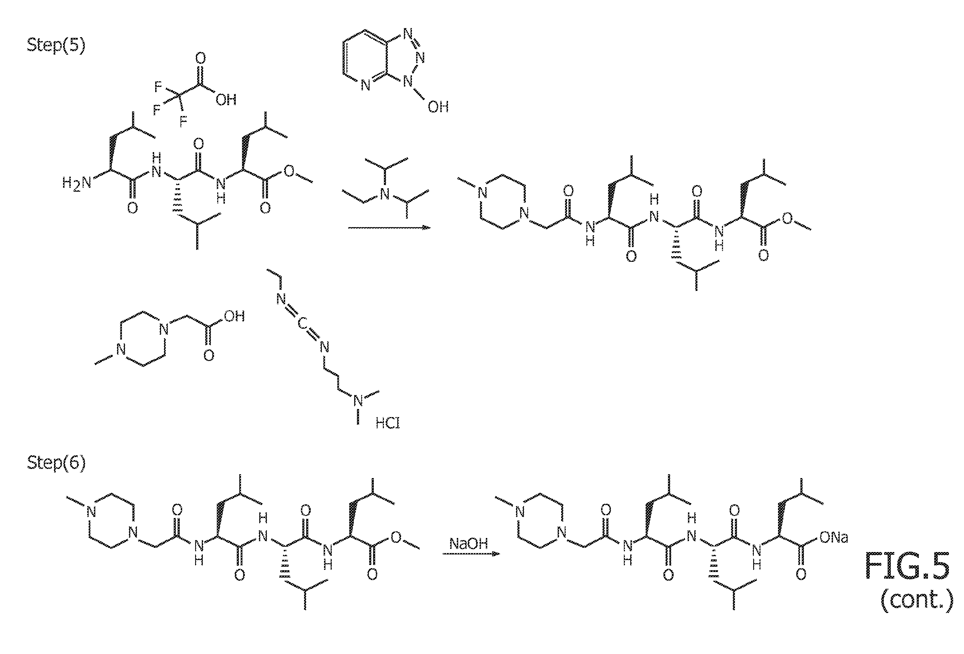

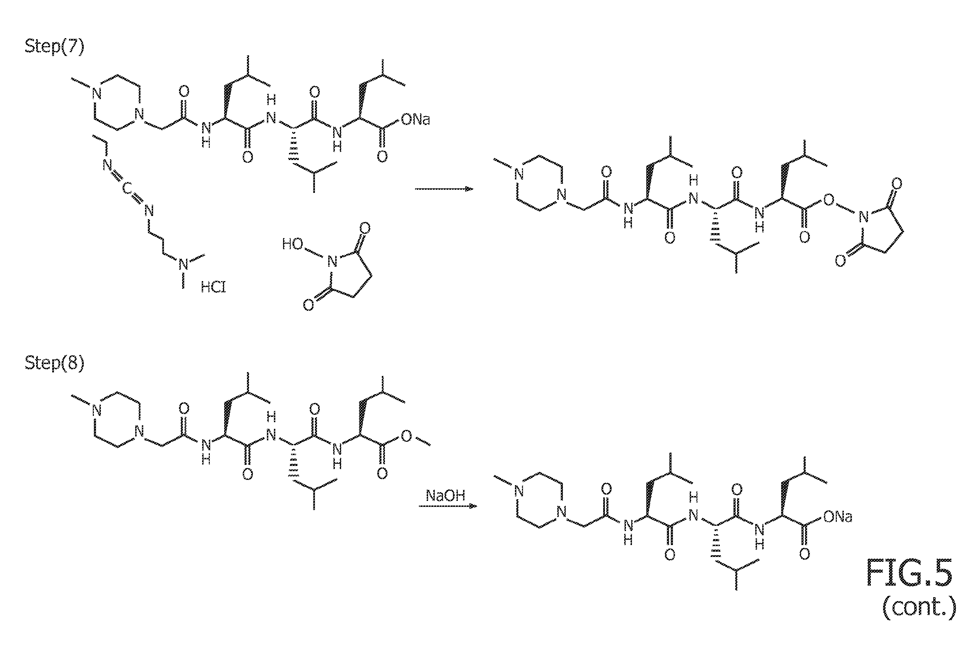

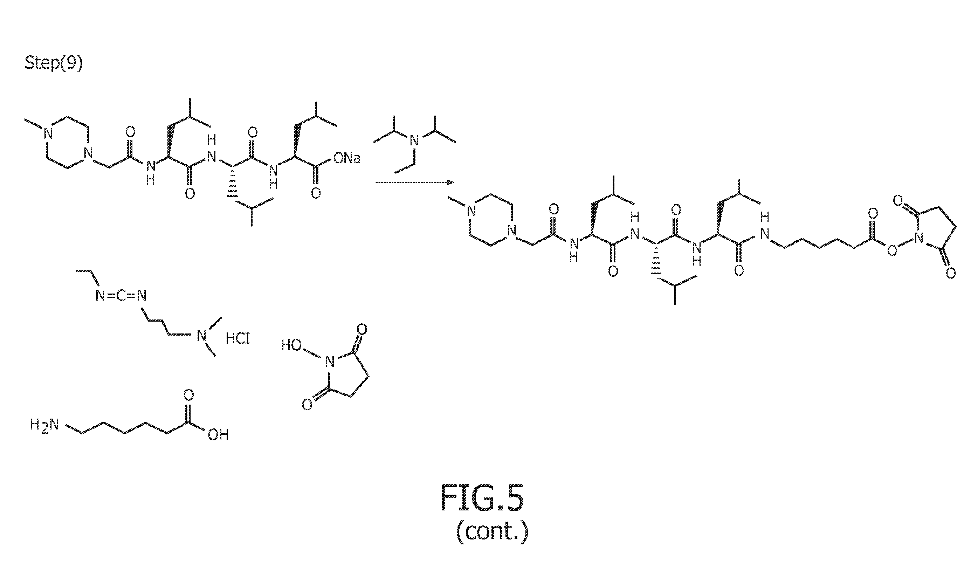

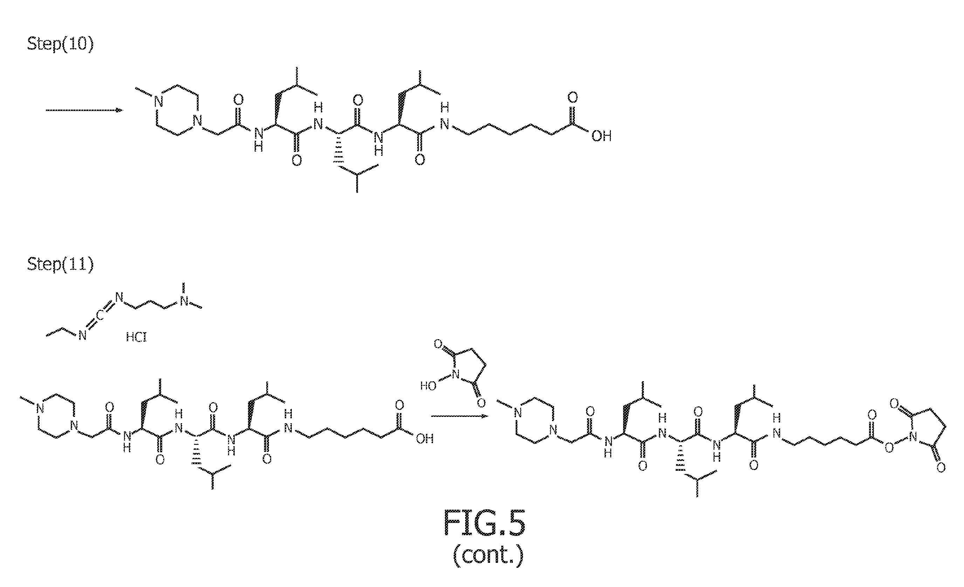

The (Leu)₃ labeling compound shown in Figs. 4A and 6A was chemically synthesized according to the following protocol. The individual synthesis steps correspond to the reaction schemes depicted in Fig. 5.

Step (1): The starting materials (S)-2-(*tert*-butoxycarbonylamino)-4-methylpentanoic acid, (S)-methyl-2-amino-4-methylpentanoate hydrochloride, 1-(bis(dimethylamino)methylene)-1H-[1,2,3]triazolo[4,5-b]pyridine-1-ium-3-oxide hexafluorophosphate (V), and N-ethyl-N-isopropylpropan-2-amine were dissolved in dichloromethane (150 ml), 2.0 ml DMF were added and the mixture was stirred for 2 h, after which the Boc protected amine had disappeared (TLC eluens EtOAc/heptane 1:1). 150 ml of saturated ammoniumchloride were added, and the crude product was extracted with dichloromethane (8 x 30 ml). The organic layers were dried over sodium sulfate and concentrated under reduced pressure. The crude product was purified over a glass filter with silicagel using 40% EtOAc in heptane as the eluens. This yielded 1.96 g (84%) of the pure product (S)-methyl-2-((S)-2-(*tert*-butoxycarbonylamino)-4-methylpentanamido)-4-methylpentanoate.

Step (2): The Boc protected dipeptide of step (1) was dissolved in DCM / TFA (44:11 ml) and stirred for 1.5 h. After working up a small sample with sat NaHCO₃ TLC showed that the reaction was complete. The reaction mixture was concentrated under reduced pressure yielding 2.58 g of a yellowish oil. ¹H NMR showed that the product must also contain some free trifluoroacetic acid along with the product, attempts to remove this by distillation failed and therefore the crude product (S)-methyl-2-((S)-2-amino-4-methylpentanamido)-4-methylpentanoate 2,2,2-trifluoracetate was used without further purification.

Step (3): The amino trifluoroacetate salt of step (2) was dissolved in dichloromethane (150 ml). At 0°C DIPEA was added, followed by HATU and Boc-Leu-OH. The mixture was stirred at room temperature for 2 h. According to TLC, the reaction was complete. The mixture was quenched with saturated ammonium chloride, and the water layer was extracted with dichloromethane (8 x 50 ml). The combined organic layers were dried over sodium sulfate and concentrated under reduced pressure. The crude product was purified over silicagel using EtOAc in heptane (4:6) as the eluens. The resulting tripeptide (6S, 9S, 12S)-methyl-6,9-diisobutyl-2,2-14-trimethyl-4,7,10-trioxo-3-oxa-5,8,11-triazapentadecane-12-carboxylate was obtained in a yield of 1.78 g (3.77 mmol; 50%).

Step (4): The Boc protected tripeptide of step (3) was dissolved in DCM / TFA (44:11 ml) and stirred for 1.5 h. After working up a small sample with saturated NaHCO₃ TLC showed that the reaction was complete (the product does not run with 50% EtOAc in heptane). The reaction mixture was concentrated under reduced pressure and stripped once with dichloromethane. Diethylether was added (20 ml) to the product for trituration, after 5 minutes, 10 ml heptane were added and the solid was filtered off after 40 minutes yielding 1,480 g of a white powder ((S)-methyl-2-((S)-2-amino-4-methylpentanamido)-4-methylpentanamido)-4-methylpentanoate 2,2,2-trifluoracetate).

Step (5): To a mixture of the tripeptide-TFA salt of step (4), N-methylpiperazinylacetic acid, EDC and HOAt in toluene (50 ml, thought it was dichloromethane) DIPEA was added, and the mixture was stirred for 1 h during which a yellow solid was formed on the bottom of the flask. The mixture was quenched with saturated sodium bicarbonate (70 ml). The two phases were separated, and the water phase was extracted with dichloromethane (4 x 50 ml). The combined organic layers were dried over sodium sulfate and concentrated under reduced pressure yielding 0.203 g of crude material, which contained both the desired product as well as the starting material (according to LC-MS and ¹H NMR). The crude material was once more treated with EDC, HOAt and DIPEA in dichloromethane (25 ml) during 2 h. After work up (similar to the above described procedure) 0.231 g of crude material was obtained. According to LC-MS the reaction was complete. Purification over silica gel with gradient from 5-10% 0.5 M NH₃ in MeOH in dichloromethane gave 0.158 g of product as a crystalline white solid ((S)-methyl-4-methyl-2-((S)-4-methyl-2-((S)-4-methyl-2-(2-(4-methylpiperazin-1-yl)acetamido)pentanamido)-pentanamido)pentanoate).

Step (6): The starting material of step (5) was dissolved in Tesser's base (dioxane/MeOH/aqueous 4 N NaOH 15:4:1), and the mixture was stirred at room temperature. After one night of stirring the solution was concentrated yielding the crude product sodium (S)-4-methyl-2-((S)-4-methyl-2-((S)-4-methyl-2-(2-(4-methylpiperazin-1-yl)acetamido)pentanamido)pentanamido)pentanoate. This compound was used in the next step without further purification Na-analysis was performed..

Step (7): To a suspension of the crude product of step (6) in dichloromethane (5 ml) at 0°C NHS and EDC were sequentially added, and the mixture was stirred at 0°C for 3 hrs. The mixture was concentrated yielding a sticky solid. The sticky solid was re-dissolved in dichloromethane (7 ml). Then, diethylether (7 ml) was added, and the solvent was evaporated until half the volume. This addition of diethylether followed by partial evaporation was repeated twice after which the precipitate (ethyl (N,N-dimethylamino)propyl-ureum and sodium chloride) was filtered. The filtrate was concentrated yielding 52 mg of a viscous oil. When looking at the ¹H NMR spectrum it seems that this fraction may contain the desired succinate ester ((S)-2,5-dioxopyrrolidin-1-yl-4-methyl-2-((S)-4-methyl-2-((S)-4-methyl-2-(2-(4-methylpiperazin-1-yl)acetamido)-pentanamido)pentanamido)pentanoate) that is contaminated among others with ethyl diisopropyl ureum. The precipitate that was filtered (33 mg) probably contains the desired product. (singlet at 2.65 ppm, succinate protons).

Step (8): The starting material of step (6) was dissolved in Tesser's base (dioxane/MeOH/aqueous 4 N NaOH 15:4:1), and the mixture was stirred at room temperature. The reaction was monitored by LC-MS (acidic mode). After 2 h of stirring the solution was concentrated yielding the crude product. This compound was used in the next step without further purification. The yield was not determined

Step (9): To a solution of crude product of step (8) in a 1:1 mixture of dioxane (dry) and dichloromethane (2.0 ml, dried) at room temperature NHS, DIPEA (26 µl) and EDC (18.9 mg) were sequentially added, and the mixture was stirred at room temperature for 5 hours. 6-amino caproic acid and 2 ml dioxane (dry) were added. The mixture was stirred overnight. On the next day, more EDC (19 mg) was added to generate the activated succinimide ester. The mixture was again stirred over night. On the next day, a sample was taken from the reaction mixture, n-butylamine was added to the sample, and the resulting mixture was stirred for 10 minutes. The solution was concentrated under reduced pressure and analyzed by LC-MS: The crude product consisted of expected butylamide from the caproic acid adduct ([M+H]+ at m/z = 666.4), the caproic acid addition product ([M+H]+ at m/z=611.4), the succinimide ester from the starting material ([M+H]+ at m/z=553.4) and its corresponding methylester ([M+H]+ at m/z=512.4). The reaction mixture was quenched in saturated sodium bicarbonate (25 ml), and water (5 ml) was added. The aqueous phase was extracted with dichloromethane (5 x 20 ml), and the combined organic layers were dried over sodium sulfate and concentrated under reduced pressure. This resulted in 41 mg of solid. The solid was analyzed with LC-MS5 to reveal that it was a mixture of compounds. A sample was taken to perform a test reaction with butyl amine (see above). The product from this test reaction was analyzed with LC-MS5. It contains the expected butyl amide but its abundance seems to be lower than in the first test reaction. Due to the complexity of the mixture and to the uncertainty about composition of the product along with its potential instability. it was decided not to ship this product.

Step (10): The product of step (9) was purified by means of preparative HPLC. This yielded 8 mg of the free acid

Step (11): To a solution of starting material of step (10) in DMF EDC and N-hydroxysuccinimide (NHS) were added, and the mixture was stirred over night under N₂ atmosphere. On the next day, more NHS (4 mg) was added, and the mixture was again stirred for 5 h. A test sample was taken and allowed to react with excess N-butylamine for analysis. LC-MS of this sample showed that the product was formed (butyl amide with m/z = 667). The mixture was concentrated under reduced pressure and re-dissolved in dichloromethane (20 ml). This solution was quenched with saturated NaHCO₃ (20 ml). The organic layer was washed with saturated NaCl and dried over sodium sulfate. A test sample was taken for analysis: Excess butylamine was added, and the mixture was stirred for 15 minutes. The solution was concentrated under reduced pressure and analyzed by LC-MS. This analysis showed that the product was of good quality. The solution was concentrated to yield 7 mg of the desired (Leu)₃ product.

### Example 3 - Isolation of phosphorylated peptides

The below example indicates how phosphorylated peptides can be identified in a complex sample. Comparable conditions can be applied to N-terminal fragments as isolated in Example 1.

Commercially available instrumentation such as the IPGPhor IEF unit (Amersham-Pharmacia, Piscataway, NJ, USA) was used for electrophoretic separation of the protein and/or peptide samples employed according to their isoelectric point. Chemical or enzymatic digestion of the proteins into peptides was optionally performed following established standard protocols.

To specifically remove the phosphate moieties from phosphoserine and phosphothreonine amino acid residues, base hydrolysis was used to induce β-elimination. In order to avoid any adverse effects on the side chains of cysteine and methionine residues, the samples were first treated with performic acid, resulting in the oxidation of these residues, thereby inactivating them (Goshe, M.B. et al.(2001) Anal. Chem. 73, 2578-2586).

The β-elimination reaction mixture used was as follows: H₂O, dimethyl sulfoxide, acetonitrile, 250 mM EDTA (pH 8.0), and 5 M NaOH. All solvents were degassed with N₂ before and after preparation of the reaction mixture.

The β-elimination reaction mixture was added to the lyophilized protein fractions isolated from the gel after isoelectric focussing. The samples were incubated for 1 h at 55°C under a N₂ atmosphere, cooled to room temperature, and the reaction quenched by neutralizing with acetic acid (Goshe, M.B. et al.(2001), *supra*)

Commercially available phosphates were to specifically dephosphorylate phospho-tyrosine amino acid residues.

By treating the protein and/or peptide samples in the above-described manner and subsequent electrophoretic separation of the respective fractions according to their isoelectric point serine/threonine-phosphorylated proteins and/or peptides as well as tyrosine-phosphorylated proteins and/or peptides could be selectively enriched and separated.

### Example 3 - MS analysis of ICAT and iTRAQ labele peptides

The below example serves to illustrate how double labeling with isobaric and isotopic labels can be used in MS analysis.

Initially, a human blood plasma sample was immuno-depleted using a Multiple Affinity Removal LC Column - Human 7 (4.6 x 10 mm; Agilent Technologies, Inc., Santa Clara, CA, USA) according to the manufacturer's instructions. Seven highly abundant plasma proteins - albumin, IgG, antitrypsin, IgA, transferrin, fibrinogen, and haptoglobin - that constitute approximately 85% of the total protein mass of human plasma were removed by this treatment. The column requires a two buffer system for operation. Buffers A and B are optimized to minimize co-adsorption of non-targeted proteins and to ensure reproducibility of column performance and long column lifetime. Buffer A - a salt-containing neutral buffer, pH-7.4 - was used for loading, washing, and re-equilibrating the column, and Buffer B - a low pH urea buffer - was used for eluting the bound high-abundant proteins from the column. The depletion was performed using an Agilent 1200 series LC system. Before injection onto the column serum from human male AB plasma, sterile-filtered (Sigma- H4522) was diluted 4X with Buffer A. The sample was transferred to a 0.22 µm spin tube and centrifuged for 1 min. at 16,000 x g to remove particulates. Diluted plasma was kept at 4°C.

The proteins were isotopically labeled using the ICAT reagent (Applied Biosystems, Inc., Foster City, CA, USA) essentially according to the manufacturer's instructions and digested with trypsin (ratio trypsin:sample = 1:20). Subsequently, the resulting peptides were isobarically labeled essentially following the established iTRAQ protocol (Applied Biosystems, Inc., Foster City, CA, USA).

However, the buffers used for ICAT are incompatible with iTRAQ labeling and *vice versa.* Since labeling and purifying the samples consecutively is time-consuming and does not take full advantage of the multiplexing procedure, the buffer composition was optimized in such way that one buffer composition can be used for both labeling reactions. Furthermore, Agilent buffer A is incompatible with iTRAQ labeling as well. Therefore, the flow-through fractions were subjected to a buffer-exchange procedure using 5K MWCO spin concentrators (Agilent Technologies) to ensure using an appropriate buffer compatible with both ICAT and iTRAQ labeling chemistries. 50 mM triethyl ammonium bicarbonate (TEAB) was found to be optimal.

The order of labeling is also important, since first performing an iTRAQ labeling without blocking cysteine amino acid residues (which are used for the ICAT labeling), results in a significantly reduced efficiency of protein digestion. The amount of starting material was 50 µg protein (as determined via a Bradford assay; Bio-Rad Laboratories, Hercules, CA, USA) dissolved in 40 µl 50 mM triethyl ammoniumbicarbonate (TEAB) (for ICAT labeling, the concentration should be 1.2 mg/ml). To each sample, 1 µl 50 mM TCEP (Tris(2-carboxyethyl) phosphine hydrochloride) and 2 µl 2% SDS were added and the mixtures were boiled for 10 minutes. Then, 20 µl acetonitrile were added to each sample. The samples were allowed to cool and each added to a vial of ICAT reagent. Incubation was done for 2 hours at 37°C.

For cleavage of the proteins, trypsin was added at a 1:20 ratio and the samples were incubate for 12-16 hours. Then, the samples were completely dried in a speed-vac. 30 µl 1M TEAB and 70 µl acetonitrile were pre-mixed per iTRAQ label and added to each ICAT labeled protein digest. Re-solubilization of the peptides should be ensured. This mixture was added to the iTRAQ reagent tube and labeled for 1 hour at room temperature. To quench the reaction, 100 µl MilliQ-purified water was added to each tube and the tubes were incubated for 30 min. at room temperature. Finally samples were dried using a centrifugal vacuum concentrator

For sample purification, to each of the sample tubes 250 µl CEX loading buffer (10 mM KH₂PO₄/25% acetonitrile (ACN), pH 3.0) were added. Samples were vortexed and sonicated during three cycles to maximize re-solubilisation of the peptides. The samples were combined and diluted to a total volume of 4 ml CEX loading buffer. Perform SCX and avidin sample clean up according to manufacturer's protocol and cleave the ICAT according to the following protocol:
- Check the pH using pH paper. If the pH is not between 2.5 and 3.3, adjust by adding more Cation Exchange Buffer-Load.
- To condition the CEX cartridge, inject 2 ml of the Cation Exchange Buffer-Load. Divert to waste.
- Slowly inject (∼1 drop/second) the diluted sample mixture onto the cation-exchange cartridge and collect the flow-through into a sample tube.
- Inject 1 ml of the Cation Exchange Buffer-Load to wash the TCEP, SDS, and excess ICAT reagents from the cartridge.
- To elute the peptides, slowly inject (∼1 drop/second) 500 µl of the Cation Exchange Buffer-Elute (10mM KH₂PO₄/ 500mM KCl / 25% ACN, pH 3.0). Capture the eluate in a fresh 1.5-mL tube. Collect the eluted peptides as a single fraction.

The purification of the biotinylated peptides and the removal of the biotin label were performed as follows:
- Inject 2 ml of the Affinity Buffer-Elute (30% ACN / 0.4% TFA ) onto the avidin cartridge. Divert to waste.
- Inject 2 ml of the Affinity Buffer-Load (2 x PBS, pH 7.2). Divert to waste.
- Neutralize each cation-exchange fraction by adding 1 ml of the Affinity Buffer-Load.
- Check the pH using pH paper. If the pH is not 7, adjust by adding more Affinity Buffer-Load.
- Vortex to mix, then centrifuge for a few seconds to bring all solution to the bottom of the tube.
- Label three fraction-collection tubes: #1 (Flow-Through), #2 (Wash), and #3 (Elute), then place in a rack.
- Slowly inject (∼1 drop/5 seconds) of the neutralized fraction onto the avidin cartridge and collect the flow-through into tube #1 (Flow-Through).
- Inject 500 µl of Affinity Buffer-Load onto the cartridge and continue to collect in tube #1 (Flow-Through)
- To reduce the salt concentration, inject 1 ml of Affinity Buffer- Wash 1 (PBS, pH 7.2). Divert the output to waste.
- To remove nonspecifically bound peptides, inject 1 ml of Affinity Buffer-Wash 2 (50mM NH₄HCO₃/20% MeOH, pH 8.3). Collect the first 500 µl in tube #2 (Wash). Divert the remaining 500 µl to waste.
- Inject 1 ml of MilliQ-purified water or equivalent. Divert to waste.
- Fill a syringe with 800 µl of the Affinity Buffer-Elute.
- To elute the labeled peptides, slowly inject (∼1 drop/5 seconds) 50 µl of the Affinity Buffer-Elute and discard the eluate.
- Inject the remaining 750 µl of Affinity Buffer-Elute and collect the eluate in tube #3 (Elute).
- Vortex to mix, then centrifuge for a few seconds to bring all solution to the bottom of the tube.
- Evaporate each affinity-eluted fraction to dryness in a centrifugal vacuum concentrator.
- In a fresh tube, prepare the final cleaving reagent by combining TFA (Cleaving Reagent A) and Cleaving Reagent B (Applied Biosystems) in a 95:5 ratio. ∼ 90 µl of final cleaving reagent per sample are required.
- Vortex to mix, then centrifuge for a few seconds to bring all solution to the bottom of the tube.
- Transfer -90 µl of freshly prepared cleaving reagent to each sample tube.
- Vortex to mix, then centrifuge for a few seconds to bring all solution to the bottom of the tube.
- Incubate for 2 hours at 37 °C.
- Centrifuge the tube for a few seconds to bring all solution to the bottom of the tube.
- Evaporate the sample to dryness in a centrifugal vacuum concentrator (∼30 to 60 min).

Multidimensional liquid chromatography (SCX-RP-LC), or alternatively isoelectric focusing of peptides followed by reverse-phase liquid chromatography (RF-LC), or 1D (RP)-LC was used to separate peptides. MALDI-MS and MALDI-MS/MS analysis were performed on 4579 spots using a 4800 MALDI-ToF-ToF analyzer (Applied Biosystems, Inc.).

**Fig.2a**shows a MS analysis of RP-HPLC fraction #12 (SCX fraction 350 mM KCl). Four ICAT peak pairs have been assigned: the expected peak ratio between ICAT(light): ICAT(heavy) = 1.8. **Fig. 2b**shows a MS/MS analysis of ICAT(light) (1655.04 Da) of peak pair #4 (see Fig. 3A). The iTRAQ reporter m/z region (114-117) is enlarged; the expected iTRAQ ratios are: 1:0.1:1:1 **Fig. 2c**shows a MS/MS analysis of ICAT(heavy) (1664.08 Da) of peak pair #4 (see Fig. 3A). The iTRAQ reporter m/z region (114-117) is enlarged; the expected iTRAQ ratios are: 1:1:1:1. **Fig. 2d**shows a MS analysis of RP-HPLC fraction #39 (SCX fraction 50 mM KCL). Four ICAT peak pairs have been assigned; the expected peak ratio between ICAT(light):ICAT(heavy) = 1.5. **Fig. 2e**shows a MS/MS analysis of ICAT(light) (1586.84 Da) of peak pair #4 (see Fig. 3D). The iTRAQ reporter m/z region (114-117) is enlarged; the expected iTRAQ ratios are: 1:0.1:1:1. **Fig. 2f**shows a MS/MS analysis of ICAT(heavy) (1595.87 Da) of peak pair #4 (see Fig. 3D). The iTRAQ reporter m/z region (114-117) is enlarged; the expected iTRAQ ratios are: 1:1:1:1.

## Claims

1. Method for the selective enrichment and/or separation of N-terminal fragments of polypeptides and/or peptides in a sample, comprising:
(a) labeling of proteins, polypeptides and/or peptides comprised within at least a first sample with at least one first label;
(b) labeling of proteins, polypeptides and/or peptides comprised within at least a second sample with at least one second label;
(c) combining the labeled proteins, polypeptides and/or peptides of step (a) and (b);
(d) digesting the labeled proteins, polypeptides and/or peptides to generate fragments thereof;
(e) fractionating said fragments;
(f) performing a methylation reaction with said fragments;
(g) re-fractionating said fragments;
(h) comparing the fractionation patterns obtained in steps (e) and (g); and
(i) separating N-terminal fragments of the polypeptides and/or peptides of steps (a) and (b) which carry a label based on the results obtained in step (g).

2. The method of claim 1, wherein said at least one label is configured to allow labeling of primary amines of said polypeptides and/or peptides.

3. The method of claim 1 or 2, wherein at least two different labels are used in steps (a) and (b).

4. The method of claim 3, wherein said at least two different labels are isotopic labels.

5. The method of claim 4, wherein said at least two different labels are isotopic labels with each label comprising further an isobaric label.

6. The method of any of claims 1 to 5, wherein any free amine groups present within said proteins, polypeptides and/or peptides are blocked before the proteins, polypeptides and/or peptides are digested such that these amine groups can not be methylated in step f).

7. The method of any of claims 1 to 6, wherein the fractionation/re-fractionation of steps (e) and (g) is performed via isoelectric focusing.

8. The method of any of claims 1 to 7, wherein the methylation reaction of step (f) allows for methylation of primary amine groups.

9. The method of claim 8, wherein the methylation reaction allows for methylation of primary amine groups at the N-termini of the fragments that are generated upon digestion in (d).

10. The method of any of claims 1 to 9, further comprising an analysis of the N-terminal fragments by means of mass spectrometry.

11. The method of any of claims 1 to 10, wherein the method includes an MS/MS scan.

12. The method of any of claims 1 to 11 wherein the method further includes the steps of:
(j) fractionating the isolated N-terminal fragments;
(k) removing or altering at least one phosphate-group from at least a first subset of N-terminal fragments;
(l) re-fractionating the isolated N-terminal fragments;
(m) comparing the fractionation patterns obtained in steps (j) and (l); and
(n) separating the at least first subset of phospho-N-terminal fragments modified in step (k) based on the results obtained in step (m).

13. The method of claim 12, wherein the phosphate-group is removed chemically via β-elimination.

14. The method of any of claims 1 to 13, wherein the method is performed in a high-throughput format.
